# EUROPEAN PATENT APPLICATION

(11) **EP 1 815 863 A1**
(43) Date of publication of application: **08.08.2007**
(21) Application number: 06075265.6
(22) Date of filing: 03.02.2006
(51) Int. Cl.: A61K 38/17, A61P 25/00, A61P 29/00

(54) **Use of TLR3 agonists for the treatment of neurodegenerative disorders**

(71) Applicant: Nederlandse Organisatie voor Toegepast-Natuurwetenschappelijk Onderzoek TNO, 2628 VK Delft (NL)
(72) Inventor: van Noort, Johannes Maria, 2332 AT Leiden (NL); Bsibsi, Malika, 2324 VK Leiden (NL)
(74) Representative: Winckels, Johannes Hubertus F.

(57) **Abstract**

The present invention relates to a method forthe treatment and/or prophylaxis of a degenerative inflammatory process in a tissue of a subject, said method comprising increasing the activity of Toll-like receptor 3 (TLR3) in cells of said tissue. In a preferred embodiment the TLR3 agonist is stathmin or stathmin-like proteins (SCG10, SCLIP or RB3).

## Description

### FIELD OF THE INVENTION

The invention relates to methods for the treatment and/or prophylaxis of degenerative inflammatory processes in a subject and to pharmaceutical preparations for use therein. The invention in a preferred aspect relates to methods for for treating neurodegenerative disorders and stimulating neuroprotective processes. The invention further relates to the use of TLR3-agonists for the preparation of a medicament for the prevention and/or treatment of an acute or chronic degenerative inflammatory process in a subject, particularly in brain or joint tissue of said subject. In a particularly preferred aspect, the invention relates to the use of stathmins for that purpose.

### BACKGROUND OF THE INVENTION

Human neurodegenerative disorders such as stroke, Alzheimer's disease and Parkinson's Disease pose an ever increasing threat to an aging population. Effective methods of treatment are lacking as is a clear understanding of the molecular mechanisms involved. Drugs currently used to treat neurological disease and injuries provide at the most a temporary relief of symptoms but do not stop or slow the underlying neurodegenerative process. Drugs that are believed to ameliorate the disease by intervening in the molecular cascades that lead to accelerated cell death or apoptosis are still under investigation.

Neurodegenerative central nervous system (CNS) disorders are associated with exaggerated local inflammatory responses that ultimately result in tissue damage. It is known that Toll-like receptors or TLR play key roles in the control of innate immune responses to pathogens in many vertebrates. TLR are transmembrane proteins with extracellular leucine-rich repeat domains, and cytoplasmic signalling domains that are similar to the cytoplasmic domain of the interleukin-1 receptor. In the human genome ten TLR family members have been identified to date. TLR1 is expressed in lymphoid organs in monocytes, leukocytes, T cells, B cells and NK cells. TLR2, TLR4 and TLR5 are widely expressed in myelomonocytic elements. Toll-like receptor 3 (TLR3) has a more restricted pattern of expression. A recent study in mice indicates that among the different TLR family members TLR3 is strikingly predominant in CNS.

TLR have been shown to recognise a wide variety of invariant pathogen-associated molecular patterns (PAMP). They mediate the production of pro-inflammatory mediators, a generally suitable response associated with the first line of defence against pathogens. Accordingly, TLR have drawn significant attention as potential targets to inhibit diseases associated with exaggerated inflammation, as targets for adjuvants to stimulate immune responses upon vaccination to prevent infectious diseases, or to promote anti-tumour immunity.

Recent data show that in particular TLR3-mediated responses can be distinct from responses mediated by other TLR family members due to a different TLR3-specific intracellular signalling pathway. In response to most PAMP TLR signalling typically activates the NF-κB-mediated pathway and leads to production of products including TNF-α, IL-1β, CXCL8 (IL-8), IL-6 and nitric oxide. Accordingly, the TLR-mediated response is generally seen as a typically antimicrobial host defence response designed to start pro-inflammatory innate immune responses and -eventually-antimicrobial adaptive immune responses.

With reference to TLR3 activation, it has been reported that TLR3 signalling in human mast cells not only fails to trigger TNF-α, IL-1β or IL-5 but instead, inhibits degranulation of these cells as well as their attachment to extracellular matrix components like fibronectin and vitronectin.

In the case of TLR3 and TLR4 ligation, production of type I interferons is also stimulated, which is generally perceived as an antiviral response since it promotes expression of major histocompatibility complex molecules and cross-presentation of cellular antigens to MHC class I-restricted cytotoxic T cells. Such a typically antiviral response appears to be mediated more efficiently by TLR3 as compared to TLR4. This particular property of the TLR3-mediated response appears to make sense in view of the fact that the only currently known agonist for TLR3 is double-stranded RNA, in many cases a structural intermediate of viral replication. It should be pointed out, however, that double-stranded RNA emerging during viral replication is generally intracellular and will only be available as an extracellular ligand upon lysis of the infected cell before viral replication is complete. As this will only be the case as an exception, current knowledge fails to fully provide a rationale for activation of extracellular, surface-exposed TLR3 as abundantly found on some cell types including fibroblasts and astrocytes.

Despite these data on the role of TLR in health and disease, it is currently not known how interfering in or controlling the activity of any TLR, either by activation or by inactivation of these receptors, could play a role in the treatment of neurodegenerative disorders.

It is an aim of the present invention to provide methods for the treatment of such disorders. It is a further aim to control local inflammatory reactions via the identification of mechanisms and targets involved in these neurodegenerative disorders and by subsequent therapeutic intervention. It is yet a further aim of the present invention to provide therapeutic compositions for the use in such methods.

### SUMMARY OF THE INVENTION

The present inventors have now surprisingly found that activation of TLR3 on human astrocytes and fibroblasts results in a repair response which consists of enhanced production of a variety of anti-inflammatory, anti-fibrotic, pro-angiogenic, chemotactic and neuroprotective mediators that -together- support regenerative responses.

In a first aspect, the present invention relates to a method for the treatment and/or prophylaxis of a degenerative inflammatory process in a tissue of a subject, said method comprising increasing the activity of TLR3 in cells of said tissue.

In addition, the present inventors have now surprisingly found that stathmin and stathmin-like proteins can act as activator or agonist for TLR3 or can activate TLR3-mediated signalling. The inventors discovered this activity in astrocytes, a major cell type in the mammalian CNS. The stathmin-activated TLR3-mediated response of astrocytes includes production of a range of neuroprotective, anti-inflammatory, angiogenic and chemotactic mediators that -together- support regenerative responses rather than polarised pro-inflammatory host-defence responses. A similar response was found for synoviocytes (joint fibroblasts) in response to TLR3 activation. Controlled TLR3 activation by stathmin or stathmin-like proteins on fibroblast-like and other cells, notably including CNS astrocytes and synoviocytes, may therefore have broad therapeutic potential in degenerative and other disorders. This is relevant to therapeutic intervention in a potentially wide range of human disorders.

In a preferred embodiment of a method of the present invention the method comprises the step of administering to the subject in need of the treatment or prophylaxis:
- a therapeutically effective amount of at least one TLR3-agonist;
- a therapeutically effective amount of at least one compound that promotes *in vivo* expression of a TLR3-agonist;
- a therapeutically effective amount of at least one compound that promotes TLR3 expression, and/or
- a therapeutically effective amount of at least one compound that promotes TLR3-mediated signalling.

In another preferred embodiment of a method of the present invention the tissue is nervous tissue, preferably brain tissue and/or synovial tissue.

In yet another preferred embodiment of a method of the present invention the cells are glial cells, preferably astrocytes, and/or synovial cells, preferably synoviocytes.

In still another preferred embodiment of a method of the present invention the degenerative inflammatory process is the result of a chronic or acute neurodegenerative disorder. The neurodegenerative disorder is preferably selected from the group consisting of stroke, hypovolemic shock, traumatic shock, reperfusion injury, multiple sclerosis, AIDS-associated dementia, neuron toxicity, Alzheimer's disease (AD), head trauma, acute spiral cord injury, Huntington's disease (HD), Parkinson's Disease (PD) and related synucleinopathies, dystonia, Tourette Syndrome, Frontotemporal Dementias (FTDs) and related tauopathies, prion disorders such as Fatal Familial Insomnia (FFI) and other disorders related to Creutzfeldt-Jakob disease, amyotrophic lateral sclerosis (ALS), trinucleotide repeat diseases, Motor Neurone disease, progressive supranuclear palsy, REM Sleep Behavior Disorder (RBD) and cancer. Particularly preferred neurogenerative disorders that may be treated or prevented by the method of the invention include stroke, multiple sclerosis, AD, PD and dementias.

Preferably the treatment in a method of the invention is accompanied by stimulation of a neuroprotective response in cells of said nervous tissue and/or wherein said treatment is accompanied by stimulation of a repair functions in cells of said synovial tissue.

The TLR3-agonist that may be used in methods of the present invention is preferably selected from the group consisting of:
- stathmin, TLR3-activating derivatives thereof and precursors thereof;
- stathmin-like proteins, TLR3-activating derivatives thereof and precursors thereof, and
- proteinaceous compounds comprising a polypeptide chain having an amino acid sequence similarity of at least 60% with an amino acid sequence selected from the group consisting of the amino acid sequences of stathmin (SEQ ID NO: 1), SCG-10 (SEQ ID NO:2), SCLIP (SEQ ID NO: 3) and RB3 (SEQ ID NO: 4).

In general, the amino acid sequence similarity relates to the similarity of the polypeptide to the complete reference sequences. In a preferred embodiment, however, the amino acid sequence similarity relates to the stathmin-like alpha helix domain corresponding to residues 44 to 138 of SEQ ID NO: 1.

The stathmin-like protein used in aspects of the present invention is preferably selected from SCG10, SCLIP and RB3; human stathmin is most preferred, and combinations of stathmins and stathmin-like proteins are also envisaged.

A method of the present invention may further comprise the use of the TLR3-agonist in combination with compounds that promote stabilisation of the stathmin-like alpha helix domain corresponding to residues 44 to 138 of SEQ ID NO: 1.

It should be noted that the method of the invention is not necessarily medical, but may also comprise non-medical embodiments. For instance, the method may be for preventing or retarding the onset of a neurodegenerative disorder in a subject, in the absence of any disorder, disease, or injury.

In another aspect, the present invention relates to a pharmaceutical composition for the treatment and/or prophylaxis of a degenerative inflammatory process in a tissue of a subject, wherein said composition comprises a therapeutically effective amount of at least one compound capable of increasing the activity of TLR3 in cells of said tissue; and b) a pharmaceutically acceptable carrier.

The pharmaceutically active compound in a composition of the invention may for instance be:
- a TLR3-agonist;
- a compound that promotes in *vivo* expression of a TLR3-agonist;
- a compound that promotes TLR3 expression, and/or
- a compound that promotes TLR3-mediated signalling.

The active compound in a preferred embodiment has the capacity of stimulating a neuroprotective response in cells of nervous tissue and/or of stimulating repair functions in cells of synovial tissue.

Highly preferred active compounds are TLR3-agonists, preferably one or more selected from the group consisting of:
- stathmin, TLR3-activating derivatives thereof and precursors thereof,
- stathmin-like proteins, TLR3-activating derivatives thereof and precursors thereof, and
- proteinaceous compounds comprising a polypeptide chain having an amino acid sequence similarity of at least 60% with an amino acid sequence selected from the group consisting of the amino acid sequences of stathmin (SEQ ID NO: 1), SCG-10 (SEQ ID NO:2), SCLIP (SEQ ID NO: 3) and RB3 (SEQ ID NO: 4).

Again, in a preferred embodiment, the amino acid sequence similarity relates to the stathmin-like alpha helix domain corresponding to residues 44 to 138 of SEQ ID NO: 1.

A composition of the invention may further comprise compounds that promote stabilisation of the stathmin-like alpha helix domain corresponding to residues 44 to 138 of SEQ ID NO: 1.

The pharmaceutical composition is meant specifically for use in methods of the invention as described above and for treatment or prevention of the disorders referred to above. In addition, the compound may be used in combination with other drugs, such as drugs that cause inflammation, in order to ameliorate the inflammatory effect of such drugs.

In still a further aspects, the present invention relates to the first and second medical indication of a TLR3-agonist selected from the group consisting of:
- stathmin, TLR3-activating derivatives thereof and precursors thereof,
- stathmin-like proteins, TLR3-activating derivatives thereof and precursors thereof, and
- proteinaceous compounds comprising a polypeptide chain having an amino acid sequence similarity of at least 60% with an amino acid sequence selected from the group consisting of the amino acid sequences of stathmin (SEQ ID NO: 1), SCG-10 (SEQ ID NO:2), SCLIP (SEQ ID NO: 3) and RB3 (SEQ ID NO: 4).

Such first and second medical indications in particular relate, respectively, to the compounds for use as a medicament and to the use of the compounds for the preparation of a medicament for the treatment and/or prophylaxis of a degenerative inflammatory process in a tissue of a subject.

It is an aspect of the present invention that the composition is for preventing or retarding the onset of a neurodegenerative disorder as described above in a subject, in the absence of any disorder, disease, or injury.

### DESCRIPTION OF THE DRAWINGS

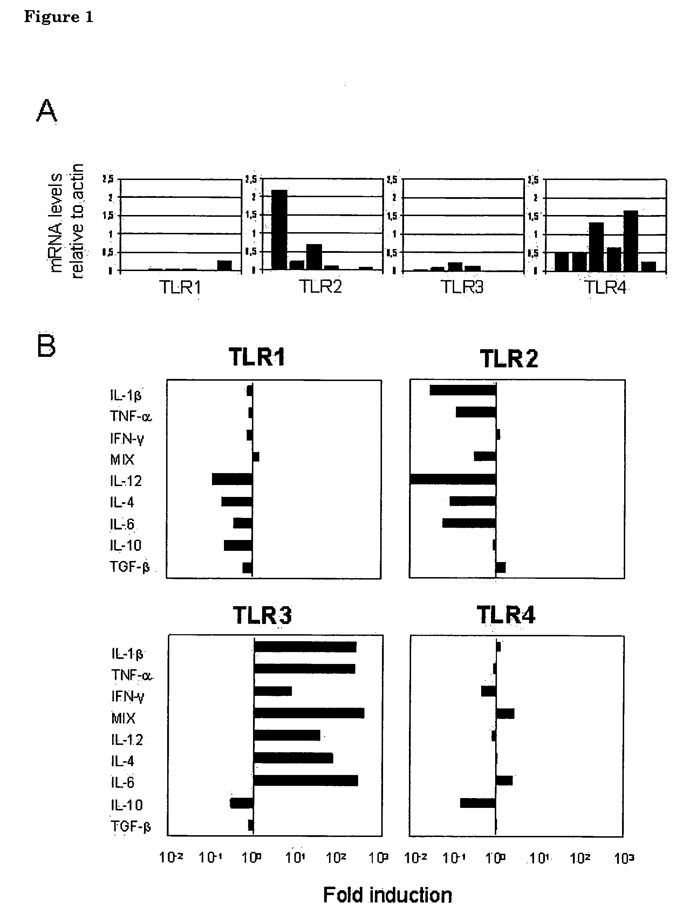

Figure 1 illustrates preferential expression of TLR4, and to a lesser extent of TLR1-3 on cultured but otherwise untreated human astrocytes from six different donors, and strong and preferential induction of TLR3 by a variety of cytokines. The data illustrate particularly strong induction of TLR3 by pro-inflammatory cytokines but not by anti-inflammatory cytokines. Panel A shows that in cultured but otherwise untreated astrocytes from six different control donors TLR4 is generally dominantly expressed, while TLR1-3 are expressed at lower levels. TLRS-10 were undetectable by RT-PCR in all cultures. Panel B shows that TLR3 is preferentially induced in human astrocytes by pro-inflammatory but not anti-inflammatory cytokines. Panel B depicts mRNA expression levels after 48 h relative to untreated control astrocyte cultures for a single representative culture. Apart from individual cytokines, also a mixture of IL-1β, TNF-α and IFN-γ (MIX) was tested.

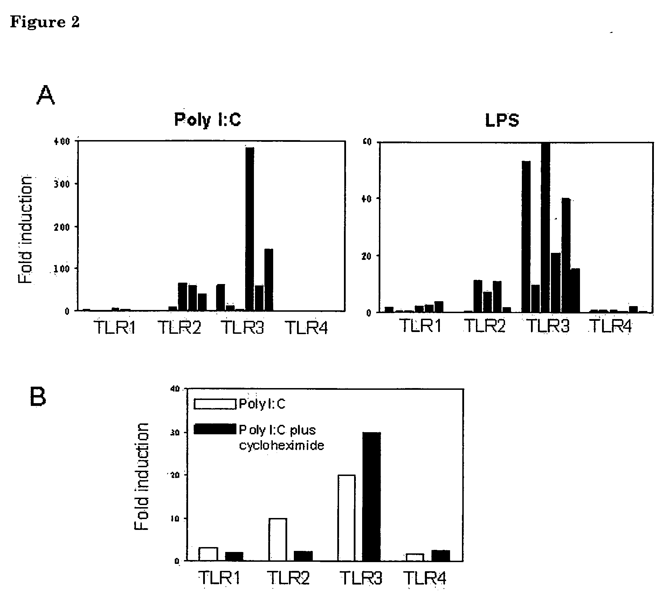

Figure 2 illustrates the preferential induction of TLR3 in cultured adult human astrocytes from six different donors under the influence of the TLR3 agonist poly I:C or the TLR4 agonist LPS. TLR3 is preferentially induced in human astrocyte cultures by TLR3 or TLR4 activation (panel A). Stimulation with poly I:C (a TLR3 agonist) or LPS (a TLR4 agonist) was performed for 24 h. The data in panel A represent individual data for 6 astrocyte cultures derived from different donors, depicting mRNA expressing levels relative to untreated control astrocyte cultures from the same donor. By activating TLR3 or TLR4, also some induction is observed of TLR2, but this is an indirect effects as it is blocked by the protein synthesis-blocking agent cycloheximide, whereas TLR3 is not blocked by such a treatment (Panel B; showing data for a single representative donor).

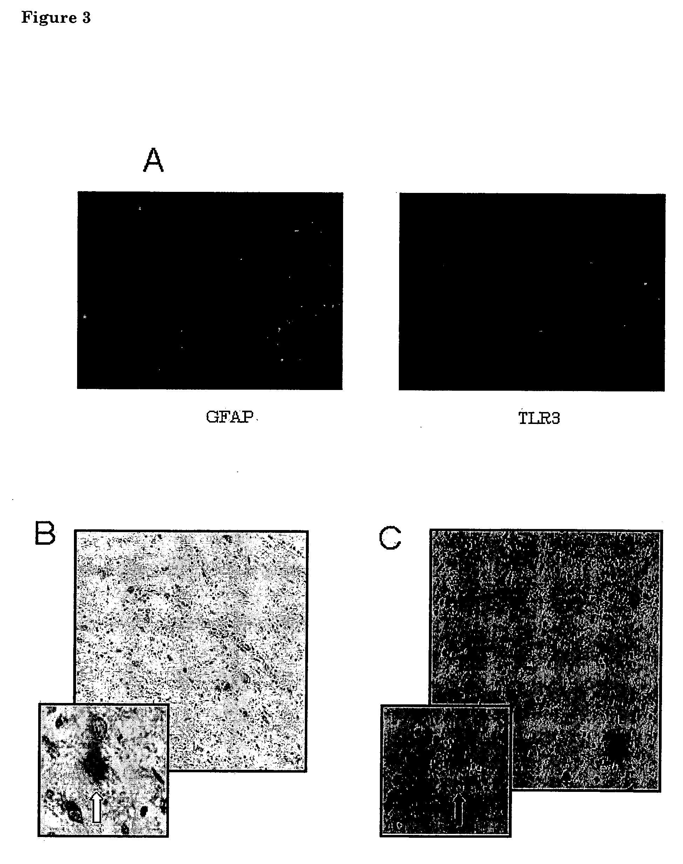

Figure 3 illustrates that in cultured adult human astrocytes, TLR3 as well as TLR4 can be found exclusively on the cell surface. TLR3 (stained by green fluoresence) is exclusively found on the cell surface of cultured GFAP-positive adult human astrocytes (panel A). TLR3 expression (stained by brown deposits) in human brain during the neuroinflammatory process of multiple sclerosis (panels B and C). Note that TLR3 staining manifests itself in two distinct ways. Astrocytes are exclusively stained in a way consistent with surface expression as found also in cell culture (panel B; open arrow), while additional vesicular staining is associated with TLR3 expressed by microglia (panel C; closed arrow), similar to the exclusively vesicular expression of TLR3 in cultured human microglia.

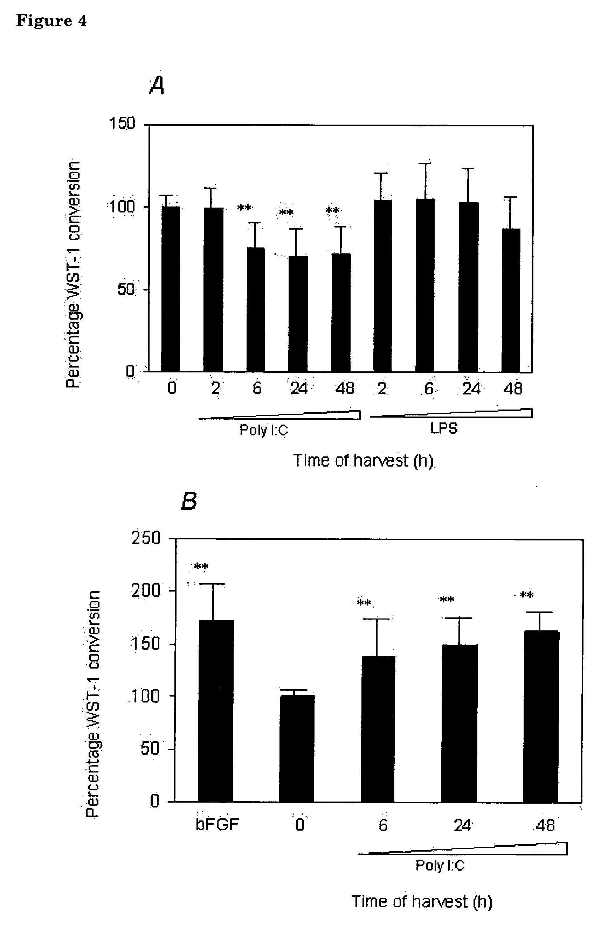

Figure 4 illustrates the effect of mediators, secreted by astrocytes in response to TLR3 activation, on growth of astrocytes (A) or endothelial cells (B). While astrocytes growth (gliosis) is attenuated by TLR3- but not TLR4-triggered mediators, endothelial cell growth is stimulated by TLR3- but not TLR4-triggered mediators. Poly I:C-conditioned medium inhibits astrocyte proliferation and promotes endothelial cell growth. Astrocytes (panel A) or endothelial cells (panel B) were cultured for 3 days in poly I:C- or LPS-conditioned astrocyte medium that had been harvested at different time points. Proliferation was evaluated by WST-1 staining of viable cells at the end of the culture period. Bars represent the pooled data ± standard deviations from six (panel A) or two (panel B) independent experiments in which each condition was assayed in quadruplicate. Significance marked ** indicates p<0.003 by analysis of variance (ANOVA).

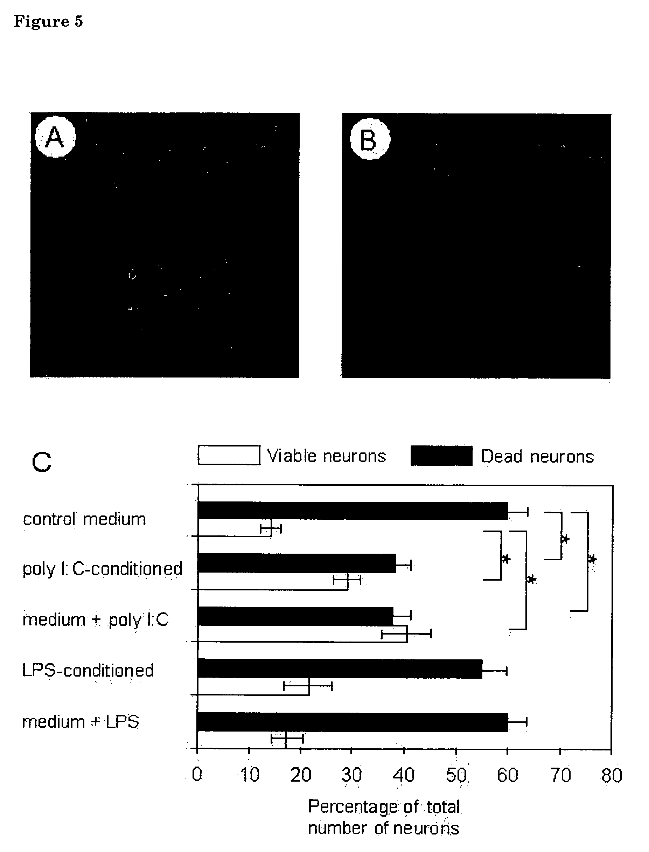

Figure 5 illustrates the survival-promoting effect on neurons in organotypic human brain slice cultures of mediators, secreted by astrocytes in response to TLR3 activation but not TLR4 activation. Poly I:C-conditioned but not LPS-conditioned astrocyte medium promotes survival of neurons in organotypic human brain slice cultures. Organotypic human cortical brain slice cultures were kept for 1 week in poly I:C- or LPS-conditioned astrocyte medium that had been harvested after 48 h. In panels A and B colour images of calcein/AM-stained brain slices are shown, illustrating markedly reduced numbers of dead cells (marked by red nuclei) in slices kept in poly I:C-conditioned astrocyte medium (panel B) as compared to control medium (panel A). In panel C percentages are given of either live or dead neurons that are the mean ± standard deviation of four individual slices examined for each condition using live/dead staining with calcein/AM. Significance marked * indicates p< 0.03 by analysis of variance (Kruskal-Wallis test).
   Note that both poly I:C-conditioned astrocyte medium and poly I:C alone significantly promotes survival of neurons relative to other culture conditions, suggesting that also when part of a culture brain slice, astrocytes activated by poly I:C can produce neuroprotective mediators.

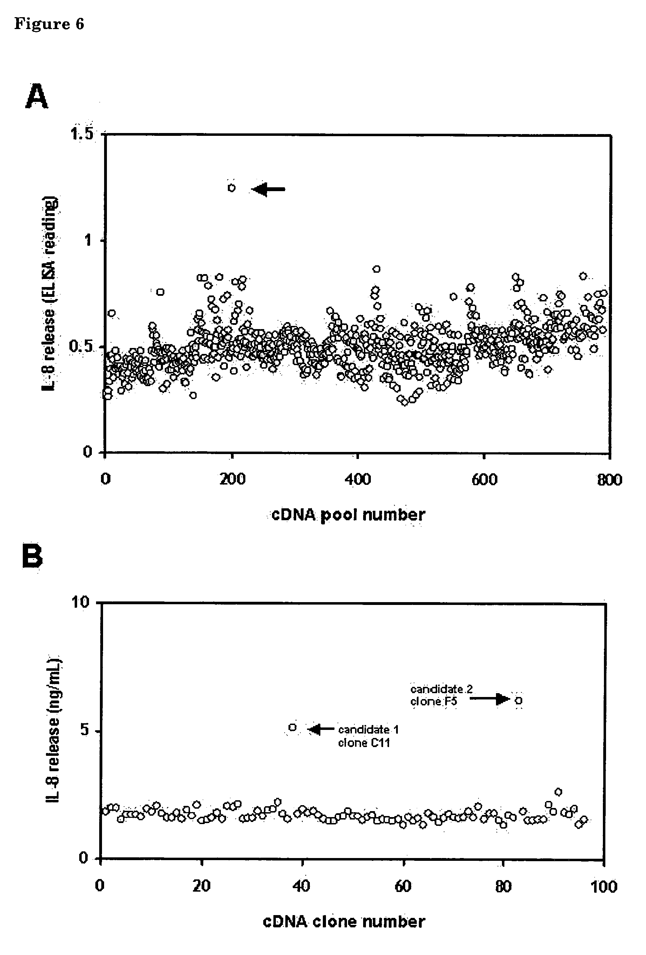

Figure 6 illustrates the results of a screening of cDNA bank-derived gene products on their ability to induce CXCL8 secretion in astrocytes, a sensitive albeit not specific marker for TLR3 engagement. This screening led to identification of a stathmin-derived sequence as one of two major CXCL8 inducing products encoded by this cDNA bank. The other product has no relevance to TLR3-mediated signalling by astrocytes. Screening of a cDNA bank derived from human brain tumours on products that trigger CXCL8 secretion by human astrocytes in culture. cDNAs were made using mRNA templates from a mixture of human brain tumour cell lines and these were individually cloned into *E. coli* using a construct that promotes expression and secretion of the product in mammalian cells. In a first step (panel A) 768 different pools of 50 to 100 individual plasmids containing the different cDNAs were transfected into COS-7 cells. Culture supernatants from those transfected cells were collected after 48 h and fed to human astrocytes in culture. After another 48 h, CXCL8 release by stimulated astrocytes was assessed by enzyme-linked immuno sorbent assays. In panel A, the results are shown for the first screening of 768 pools of cDNA, illustrating that one pool (marked by an arrow) induced markedly higher than average CXCL8 release by astrocytes. Subsequent testing of all individual cDNAs contained in that pool (panel B) reveals two cDNA candidate agonists. Candidate 1 was found to be irrelevant to the current invention while candidate 2 (clone F5) contains the sequence corresponding to residues 43-149 of human stathmin.

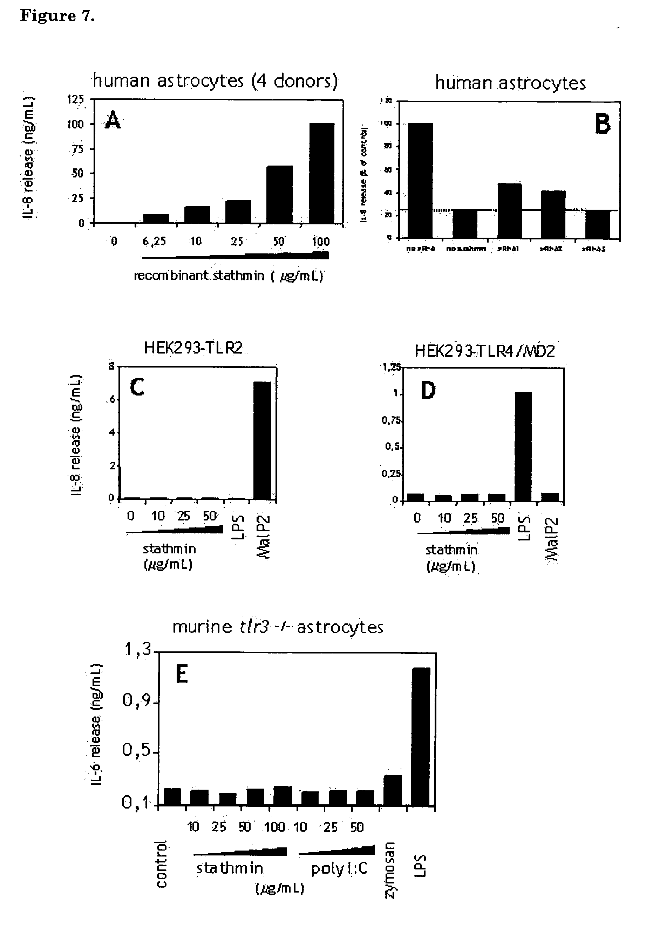

Figure 7 illustrates the ability of stathmin to induce secretion of CXCL8 (interleukin-8; referred to as IL-8) in human astrocytes or IL-6 in murine astrocytes, sensitive albeit not specific markers for TLR3 engagement, but the failure of stathmin to do so in astrocytes isolated from TLR3-deficient mice, confirming the notion that the astrocyte response to stathmin is TLR3-mediated. Also shown is the failure of stathmin to activate either TLR2 or TLR4, ruling out any confounding effects by contaminant endotoxins in the stathmin preparation. Activation of astrocytes by recombinant human stathmin is TLR3-dependent.
   In Panel A, the averaged response of 4 individual human adult astrocyte cultures is shown to increasing doses of recombinant stathmin; release levels of CXCL8 in response to poly I:C in these experiments is around 200 ng/mL. Possibly due to the fact that only part of the stathmin molecules in solution adopt the full alpha helical conformation required for TLR3 binding, the astrocyte response to stathmin is still not maximal at 100 µg/mL. In panel B the effect of three different small interfering RNAs (siRNA) specific for human TLR3 are shown, revealing that the stathmin-induced CXCL8 response in human astrocytes is strongly reduced or even fully ablated by these siRNAs. In panels C and D, it is shown that at levels of at least 50 µg/mL the preparation of recombinant stathmin used does not contain any endotoxin contaminants that trigger responses of HEK293 transfectant cells either via TLR2 (panel C) or via TLR4 (panel D). In Panel E, it is shown that for astrocytes of TLR3-deficient mice, both the stathmin- and the poly I:C-induced response is ablated, while the response to LPS (TLR4 agonist) is still intact.

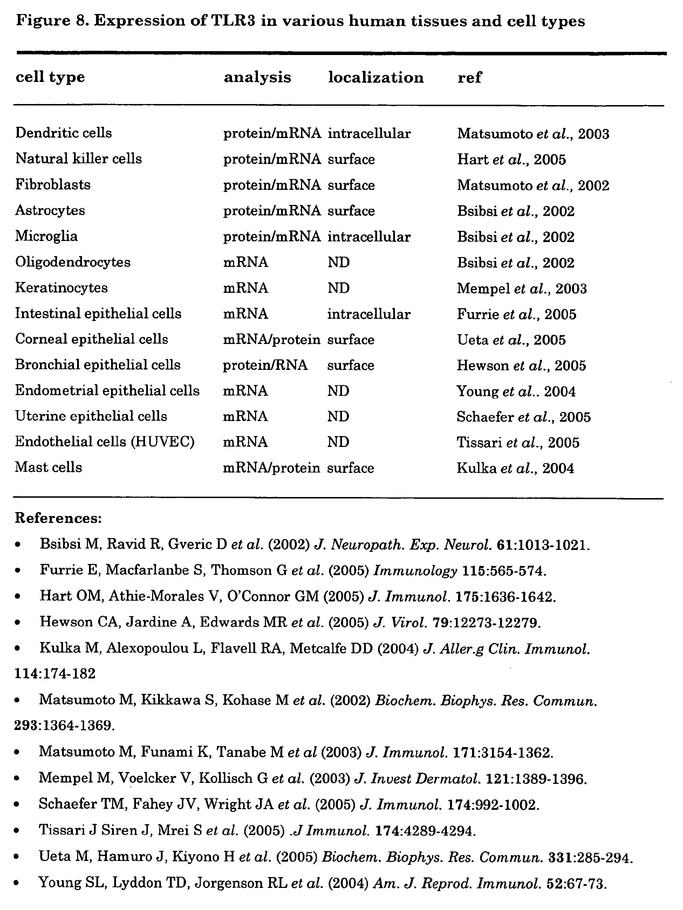

Figure 8 summarises the currently documented expression of TLR3 in different human tissues and cell types under normal (non-diseased) conditions.

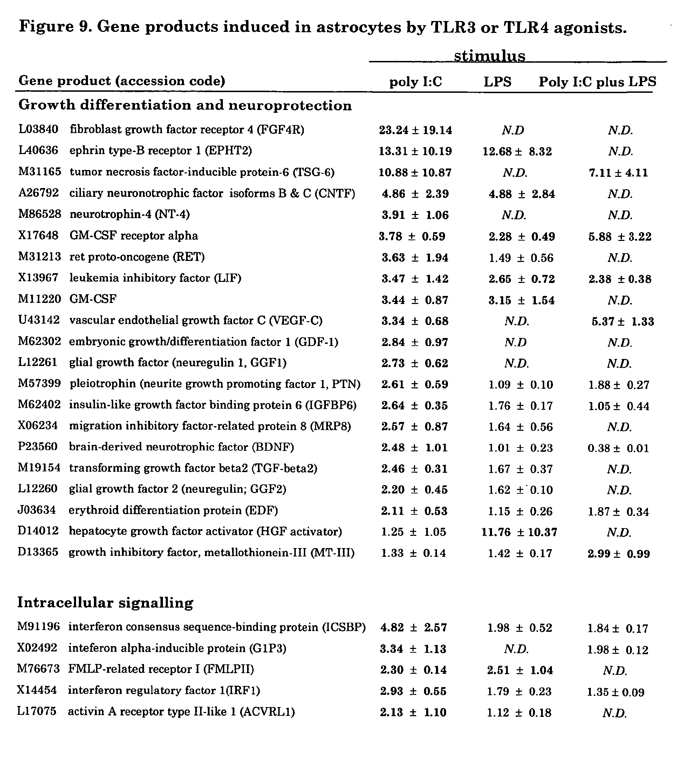

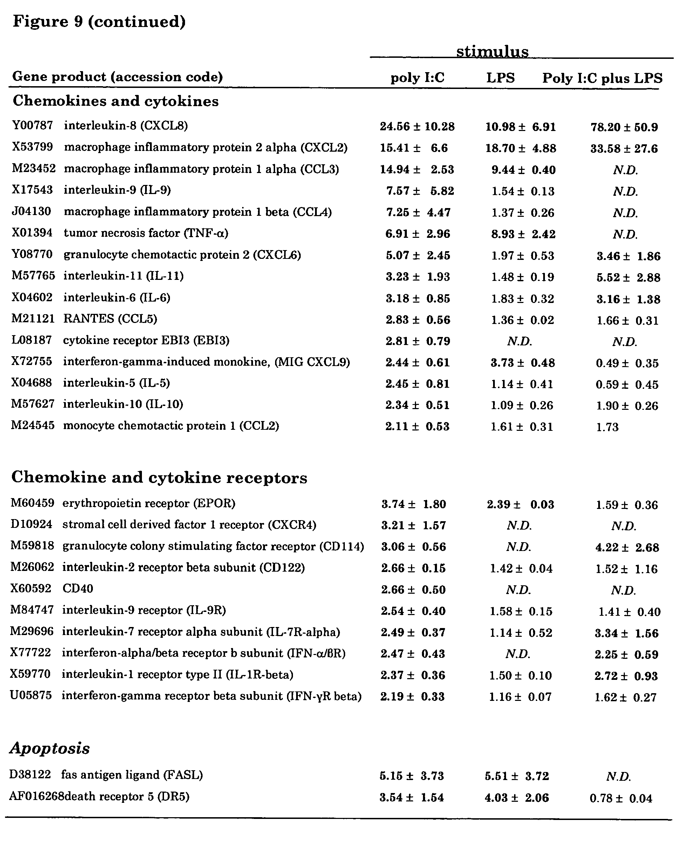

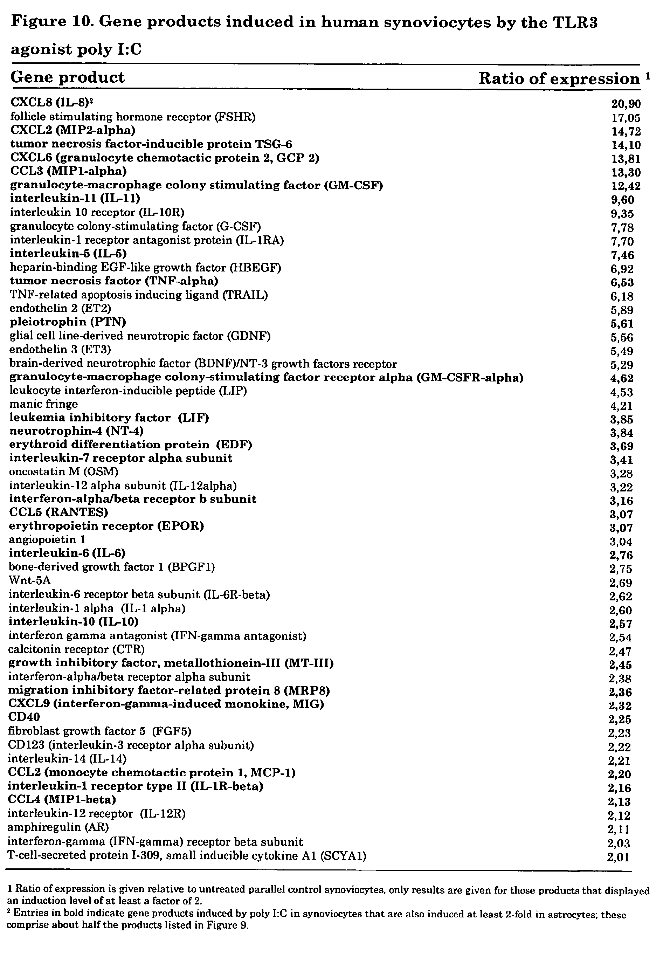

Figure 9 lists all gene products represented on a macroarray used in the example study whose expression levels were found to be reproducibly increased at least two-fold following stimulation of astrocytes with either the TLR3 agonist poly I:C, the TLR4 agonist LPS or the mixture of both. Numerical values represent the level of mRNA expression in the stimulated astrocyte population relative to their expression in unstimulated astrocytes from the same donor, cultured for the same time under the same culture conditions but without the specific TLR agonist. The numerical values represent the maximum stimulation found after analysing levels of expression after 2, 6, 24 and 48 h. Ratios are given of the gene expression signals found in agonist-treated astrocytes cultures relative to untreated control cultures after a 48-h culture period. Data represent average ratios ± standard deviation for 4 cultures derived from different donors. Only data are given for those gene products that show at least 2-fold induction (in bold) under any of the three experimental conditions. ND: not reliably detectable; average gene expression signal below 10% threshold relative to housekeeping gene expression signals. Note that about half of the mediators induced by poly I:C in astrocytes are also induced by poly I:C in human synoviocytes (see Figure 10). Figure 10 lists all gene products induced by poly I:C in cultured human synoviocytes

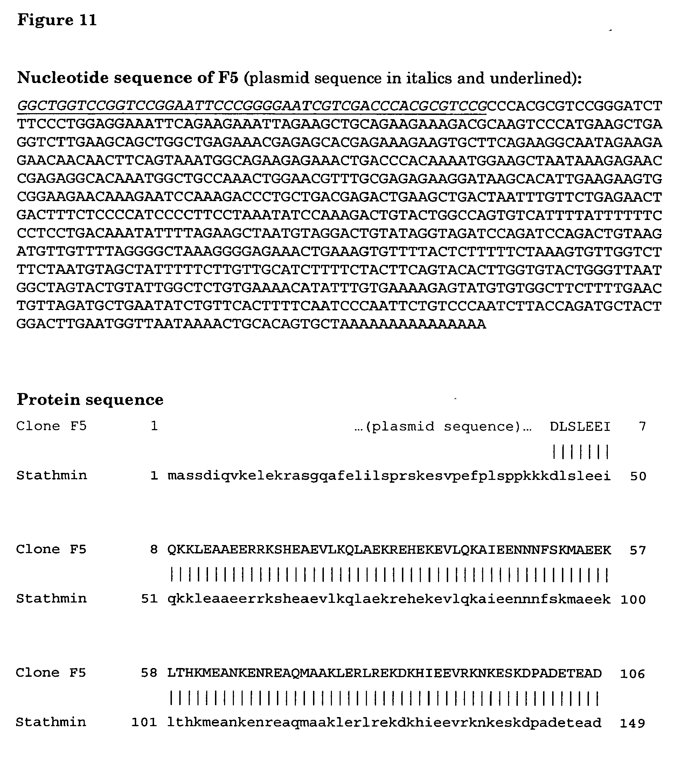

Figure 11 shows the nucleotide sequence of the cDNA clone (clone F5) found in the screening experiments described below in the experimental section and its corresponding protein sequence encoding a protein capable of activating TLR3-mediated signalling. The protein sequence encoded by F5 (excluding the plasmid sequence) is identical to residues 43 (D, aspartic acid) to the C-terminal residue 149 (D, aspartic acid) of human stathmin, which stretch fully comprises the conserved stathmin-like alpha helical domain that is located between residues 44 and 138.

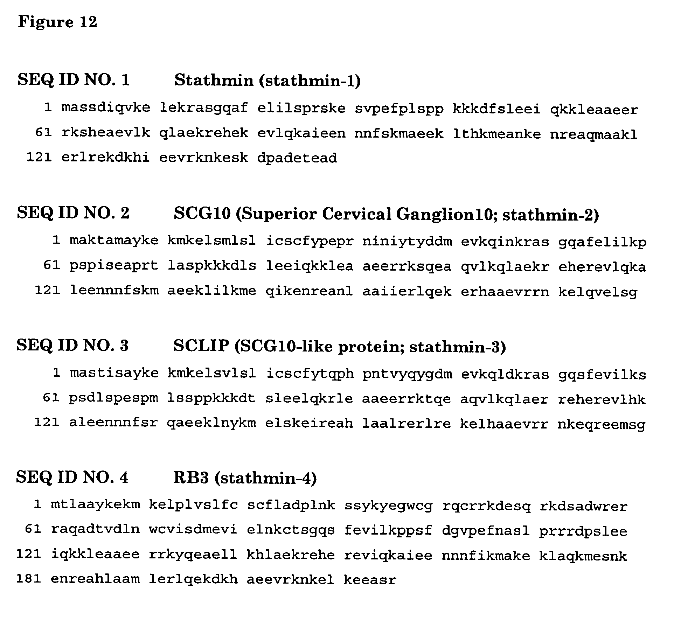

Figure 12 provides the amino acid sequences of all four currently known human members of the family of stathmin-like proteins.

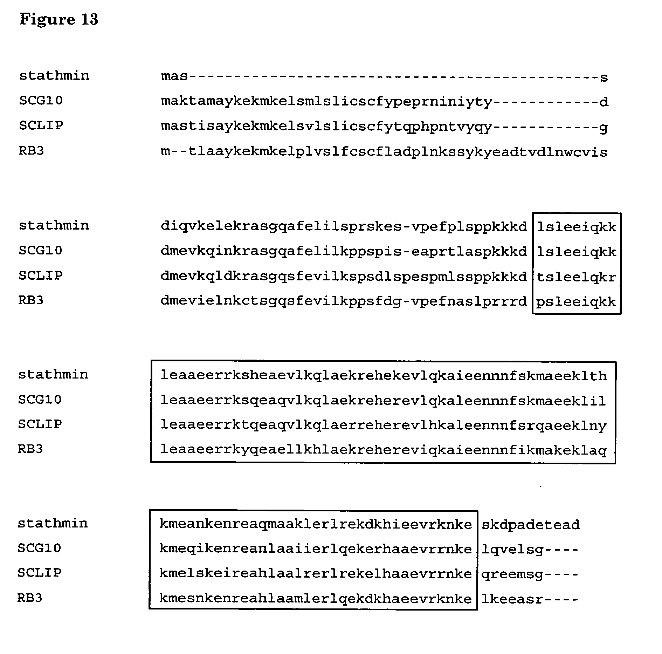

Figure 13 highlights the sequence homologies within the conserved stathmin-like alpha helical domains of the four human stathmin-like family members stathmin, SCG10, SCLIP and RB3. Sequence similarities among the four family members of human stathmin-like proteins. Note that most of the sequence differences reside in the N-terminal domain that controls intracellular trafficking and subcellular localisation. The highly conserved stathmin-like alpha helix domain corresponding to residues 44 to 138 of stathmin is boxed. The percentage amino acid identity of this helical part of the sequence of stathmin with the other family members is between 73 and 82.

### DETAILED DESCRIPTION OF THE INVENTION

### Definitions

"Treatment and/or prophylaxis" as used herein includes retarding and/or reversing the progression of tissue deterioration or destruction, as well as inhibiting or preventing such deterioration or destruction and reversing the degradation process into a regeneration process. In case the deterioration or destruction takes place or is directed at nervous tissue, the treatment and/or prophylaxis may involve neuroprotective processes as described herein. As used herein the term is specifically used in connection with the TLR3-mediated repair response which consists of enhanced production of a variety of anti-inflammatory, anti-fibrotic, pro-angiogenic, chemotactic and neuroprotective mediators that -together- support regenerative responses. The term "regenerative responses" indicates the opposite of tissue degeneration processes, i.e. it indicates the tissue regeneration process, including the provision of protection against cell damage. Thus, when the term "TLR3-mediated repair response" is used herein it is meant to indicate all of the above effects.

As used herein the terms "tissue degeneration", "tissue deterioration" or destruction" are used interchangeably and describes any internal or external process which causes damage or injury to a tissue or an impaired function of a body tissue of tissue failure, which may be characterised by impaired cellular activity or death of cells in the tissue and tissue necrosis, and includes in general histological alteration. This degeneration can be caused by medication, by viral infection, by trauma, by anoxia or by starvation or any other process or disorder that results in chronic or acute degeneration of cells in the tissue or in a supporting tissue, such as by the detrimental effect of exaggerated inflammation.

As used herein, the term "neurodegenerative disorder", including chronic and acute neurodegenerative disorders such as stroke, hypovolemic shock, traumatic shock, reperfusion injury, multiple sclerosis, AIDS-associated dementia, neuron toxicity, Alzheimer's disease (AD), head trauma, acute spiral cord injury, Huntington's disease (HD), Parkinson's Disease (PD) and related synucleinopathies, dystonia, Tourette Syndrome, Frontotemporal Dementias (FTDs) and related tauopathies, prion disorders such as Fatal Familial Insomnia (FFI) and other disorders related to Creutzfeldt-Jakob disease, amyotrophic lateral sclerosis (ALS), trinucleotide repeat diseases, Motor Neurone disease, progressive supranuclear palsy, REM Sleep Behavior Disorder (RBD) and cancer.

A "neuroprotective" effect is defined herein as an inhibition, prevention or generally control of processes that cause damage or injury to cells of the nervous tissue, in particular to the neurons. The neuroprotective effect of TLR3 stimulation by stathmin was observed in astrocytes as described in more detail in the Examples below.

The term "nervous tissue" is used herein in it's the art-recognized meaning of a tissue composed of impuls-conducting neurons (ganglion cells) and their supporting glial cells. The supporting glial cells cells of the nervous system include for instance the capsule or satellite cells of peripheral ganglia, ependyma, neuroglia (astrocytes, oligodendroglia and microglia), and Schwann cells.

Stathmin, originally also named oncoprotein 18 (or Op-18), is a cytosolic phosphoprotein originally characterized as an intracellular relay molecule that integrates various signalling pathways. Combinatorial phosphorylation of four serine residues in the N-terminal region of the molecule is believed to affect at least part of its functions. A major function of stathmin is regulation of microtubule assembly.

As used herein, the term "stathmin-like protein" (*sensu stricto*) refers to the art-recognized term for a particular group of proteins that has amino acid sequence motives in common with stathmin (stathmin-1) and is generally used to address the group of compounds consisting of the stathmin-like proteins SCG10 (Superior Cervical Ganglion 10; stathmin-2), SCLIP (SCG10-like protein; stathmin-3), and RB3 (stathmin-4). The term "stathmin-like protein" is also used herein to refer to a proteinaceous compound comprising a polypeptide chain having an amino acid sequence similarity of at least about 60%, preferably at least 70%, more preferably at least 80%, still more preferably at least 90%, even more preferably at least 95%, and particularly preferably at least 97%, 98% and most preferably at least 99%, with an amino acid sequence selected from the group consisting of the amino acid sequences of stathmin (SEQ ID NO: 1), SCG-10 (SEQ ID NO:2), SCLIP (SEQ ID NO: 3) and RB3 (SEQ ID NO: 4). Preferably the amino acid sequence similarity relates to a region of at least 30 contiguous amino acids, preferably at least 50, more preferably at least 80 and most preferably at least 90 contiguous amino acids. Most preferably the amino acid sequence similarity relates to the stathmin-like alpha helix domain corresponding to residues 44 to 138 of human stathmin (SEQ ID NO: 1), resulting in a stathmin-like protein wherein the basic structural features consistent with the stathmin-like alpha helical domain that allows interaction with TLR3 is maintained.

The term "amino acid sequence similarity" as used herein denotes the presence of similarity between two polypeptides or proteins. Polypeptides have "similar" sequences if the sequence of amino acids in the two sequences is the same when aligned for maximum correspondence. Sequence comparison between two or more polypeptides is generally performed by comparing portions of the two sequences over a comparison window to identify and compare local regions of sequence similarity. The comparison window is generally from about 10 to 100 contiguous amino acids. The "percentage of sequence similarity" for polypeptides, such as 50, 60, 70, 80, 90, 95, 98, 99 or 100 percent sequence similarity may be determined by comparing two optimally aligned sequences over a comparison window, wherein the portion of the polypeptide sequence in the comparison window may include amino acid deletions, modification or addition of single amino acids or groups of amino acids as compared to the reference sequence (which does not comprise additions or deletions) for optimal alignment of the two sequences. The percentage is calculated by: (a) determining the number of positions at which the identical amino acid occurs in both sequences to yield the number of matched positions; (b) dividing the number of matched positions by the total number of positions in the window of comparison; and (c) multiplying the result by 100 to yield the percentage of sequence similarity. Optimal alignment of sequences for comparison may be conducted by computerized implementations of known algorithms, or by visual inspection. Sequence comparison and multiple sequence alignment algorithms are readily available on the internet, for instance via the National Center for Biotechnology Information, U.S. National Library of Medicine, 8600 Rockville Pike, Bethesda, MD 20894.

A "derivative", "analog" or "analogue" is defined herein as a stathmin or stathmin-like protein as defined herein that is subjected to a (bio)chemical modification (e.g. organo-chemical or enzymatical). Derivatising may comprise the substitution of certain chemical groups to the protein, e.g. phosphorylation, thereby retaining the TLR3-agonist character of the compound. Such derivatizations are known in the art. The derivatives and analogues maintain the biological activity of the natural stathmin or stathmin-like protein and act in a comparable way, but may provide advantages to the natural molecule such as longer half-life, resistance to degradation or an increased activity. Conservative amino acid substitutions may be made, for example within the group of aliphatic non-polar amino acids (Gly, Ala, Pro, Ile, Leu, Val), the group of polar uncharged amino acids (Cys, Ser, Thr, Met, Asn, Gln), the group of polar charged amino acids (Asp, Glu, Lys, Arg) or the group of aromatic amino acids (His, Phe, Tyr, Trp). Stathmin-like proteins of the invention may be chemically modified, e.g. post-translationally modified. For example, they may be glycosylated or may comprise modified amino acid residues. They may also be modified by the addition of histidine residues or an epitope tag for example by a repeat of several His residues or other sequence tags well known to those skilled in the art to assist their purification or detection. They may be modified by the addition of a signal sequence to promote insertion into the cell membrane. Such modified proteins also fall within the scope of the term "stathmin-like proteins, or derivatives or precursors thereof'.

A "precursor" is defined herein as a derivative of the active compound with similar, less or no activity, and which can be transformed to the active compound e.g. by the digestive tract or other digestive systems in the body. Such precursors can be obtained by chemical or enzymatic modification of the active molecule.

"Subject" as used herein includes, but is not limited to, mammals, including, e.g., a human, non-human primate, mouse, pig, cow, goat, cat, rabbit, rat, guinea pig, hamster, horse, monkey, sheep, or other non-human mammal; and non-mammal animals, including, e.g., a non-mammalian vertebrate, such as a bird (e.g., a chicken or duck) or a fish, and an invertebrate. A preferred subject in aspects of the invention is a mammal, most preferably a human.

The terms "pharmaceutical compound", "(pharmaceutically) active compound", "(pharmaceutically) active substance", "therapeutic agent", "pharmaceutical agent", "medicament" and "drug" are used herein interchangeably.

The term "pharmaceutically acceptable carrier" refers to a carrier for administration of the therapeutic agent. The term refers to any pharmaceutical carrier that does not itself induce the production of antibodies harmful to the individual receiving the composition, and which may be administered without undue toxicity. As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the stathmin or stathmin-like protein. Suitable pharmaceutically acceptable carriers in therapeutic compositions may be liquids such as water, saline, glycerol and ethanol. Liposomes are included within the definition of a pharmaceutically acceptable carrier. Suitable carriers may also be large, slowly metabolized macromolecules such as proteins, polysaccharides, polylactic acids, polyglycolic acids, polymeric amino acids, amino acid copolymers, and inactive virus particles. Such alternative carriers are also well known to those of ordinary skill in the art. Additionally, auxiliary substances, such as wetting or emulsifying agents, pH buffering substances, and the like, may be present in such vehicles. A thorough discussion of pharmaceutically acceptable excipients is available in Remington's Pharmaceutical Sciences (Mack Pub. Co., N.J. 1991).

The term "therapeutically effective amount" as used herein refers to an amount of a therapeutic agent to treat, ameliorate, or prevent a disease or condition, or to exhibit a detectable therapeutic or prophylactic effect. The precise effective amount needed for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation.

TLR3 is identified herein as a preferred mediator of tissue repair responses in mammalian cell types such as for instance astrocytes and synoviocytes. The TLR3-mediated repair response consists of enhanced production of a variety of anti-inflammatory, anti-fibrotic, pro-angiogenic, chemotactic and neuroprotective mediators that -together- support regenerative responses rather than polarised pro-inflammatory host-defence responses.

TLR3 is localised in intracellular vesicles in dendritic cells and CNS microglia, while it is exclusively found as a surface-exposed membrane protein in fibroblasts and astrocytes. When intracellular, TLR3 resides in multivesicular bodies associated within the endosomal/lysosomal pathway of endocytosis and phagocytosis. To what extent such differences in localisation relate to different TLR functions and may be associated with different functional responses to TLR agonists remains to be established. One hint that this might be the case has emerged from a recent study that shows TLR3 signalling to become augmented by an acidic environment, and also requires multimerisation of TLR3 molecules. To certain agonists, therefore, a TLR3-mediated response may be affected by its presence either in acidic endosomal vesicles or expression at the cell surface.

TLR3 ectodomain is a horseshoe-like structure assembled from 23 leucine-rich repeats that is partly glycosylated. Highly conserved TLR3-specific structures exists that are consistent with ectodomain dimer formation. Patches of positively charged amino acid residues are present on the surface of the TLR3 ectodomains that face each other in the dimer configuration, and these can be considered suitable contact regions for an extended rod-like double-stranded RNA helix lined with negatively charged phosphate groups.

The present inventors have discovered that activation of TLR3 or promotion of the expression of TLR3 or promotion of the TLR3 signalling pathway results in retarding of neurodegenerative processes. In attempting to identify a therapeutically useful agonist for TLR3 it was surprisingly found that stathmin and stathmin-like proteins provide very suitably such agonists.

In testing the efficacy of the agonist the inventors have found that activation of the TLR3-mediated response leads not only to the activation of said repair responses but also to enhanced and selective further expression of TLR3 itself. This renders the TLR3-mediated response self promoting.

Interestingly enough, the use of stathmin as an agonist has further revealed to also enhance and selectively further express stathmin itself, again rendering the stathmin-induced TLR3-mediated response self promoting.

The present invention further relates to a method for retarding or inhibiting an acute or chronic degenerative inflammatory process and/or for stimulating a neuroprotective process in a mammal in need thereof, preferably a human, comprising administering to said mammal a therapeutically effective amount of a stathmin or stathmin-like protein.

As disclosed herein TLR3-activating substances, or of another substance that specifically promotes expression of and/or functional interactions between stathmin or stathmin-like proteins and TLR3 are effective against autoimmunity, transplant or graft rejection, inflammation, allergic responses, other autoimmune, immune-mediated or trauma related tissue degeneration tissue diseases and exaggerated inflammation. Thus, the present invention discloses methods of use for permitting or promoting wound healing; controlling neurodegenerative disorders, including damage or injury to neurons.

That controlled TLR3 activation by stathmin or stathmin-like proteins on fibroblast-like and other cells is relevant to therapeutic intervention in a potentially wide range of degenerative and other human disorders may be concluded from three key observations:
- First, it was found that cultured adult human astrocytes have a remarkable preference to express elevated levels of TLR3 when activated in different ways, notably including TLR3 engagement itself. Supplying pro-inflammatory cytokines, TLR3 or TLR4 agonists or applying oxidative stress generally led to strong induction of TLR3 expression on human astrocytes and much less so, if at all, of other TLR family members.
- Secondly, a cDNA array-based analysis of the response by cultured human adult astrocytes to TLR3 activation revealed that such activation (but not TLR4 activation) induces a wide range of well-known anti-inflammatory, neuroprotective, angiogenic and chemotactic mediators. In functional assays it was verified that, together, these TLR3-triggered factors promote survival of human neurons in brain slice cultures, stimulate endothelial cell growth and inhibit astrocyte growth (gliosis). Thus, TLR3-mediated signalling by astrocytes appears functional in promoting repair and regeneration rather than initiating a pro-inflammatory host-defence response. A similar response was observed in human synoviocytes.
- Thirdly, in search of a potential endogenous TLR3 agonist by screening gene products produced by human brain tumours the inventors surprisingly identified a sequence of stathmin as candidate TLR3 agonist. The ability of this gene product to activate astrocytes was mimicked by full-length recombinant human stathmin and demonstrated to be TLR3-dependent by showing the failure of stathmin to activate astrocytes from TLR3-deficient mice and ruling our TLR2- or TLR4-mediated effects. Thus, stathmin and stathmin-like proteins activate TLR3-mediated signalling at least in astrocytes, and in doing so activate the production of a range of anti-inflammatory and tissue-repair mediators. The data also suggest that the response of TLR3 to stathmin is at least to some extent self-amplifying since TLR3-mediated signaling induces higher levels of TLR3 itself and promotes stathmin expression in different neural cell types.

Stathmin is the founding member of a family of four phosphoproteins that share a conserved so-called stathmin-like alpha helical domain of 94 amino acids containing as many as 24 negatively charged amino acids. Stathmins play key roles in the regulation of microtubule formation and they are expressed at relatively high levels by both neurons and glial cells in the CNS. The current invention discloses that stathmin acts as an agonist for TLR 3, a TLR family member that so far has been known to act as a receptor for double-stranded RNA only. When activated on human fibroblast-like cells, in particular on astrocytes of the CNS, TLR3 mediated signalling leads to production of a variety of mediators with neuroprotective, anti-inflammatory, angiogenic and chemotactic functions. A similar response is disclosed for human synoviocytes, fibroblast-like cells that are important in the pathogenesis of rheumatoid arthritis. It is also known that TLR3 mediates anti-allergic responses in human mast cells. The invention thus reveals that by activating the said repair-like response mediated by TLR3 stathmin and stathmin-like proteins may have broad therapeutic potential in CNS disorders, arthritis, allergies and other disorders involving acute or chronic tissue degeneration.

Stathmin is the generic element of a phosphoprotein family, including the proteins SCG10 (Superior Cervical Ganglion 10 protein; stathmin-2), SCLIP (SCG 10-like protein; stathmin-3), and RB3 (stathmin-4) that are highly conserved amongst vertebrates. For example, the human stathmin sequence of 149 amino acids differs from the rat sequence by only a single amino acid, and from murine stathmin by only two residues. Different members of the stathmin-related family of proteins contains variable N-terminal sequences that are relevant to intracellular targeting, in particular with regard to interactions with membranes. The common feature of the family of stathmin-like proteins is the conserved stathmin-like domain, an unusually long alpha helical structure that interacts with tubulin. As a consequence, stathmin family members also share the functional activity of regulating microtubule dynamics. It is tempting to speculate that the conserved very long C-terminal alpha helix of stathmin and stathmin-like proteins is the key structural feature that allows members of this structural family to interact with the ectodomain of TLR3. This continuous helix extends in the human stathmin sequence from residue 44 to residue 138. Thus, it extends over about 95 amino acids forming an only slightly bent rod-like structure of about 140 Å in length. Especially since negatively charged amino acids are abundant in this stathmin-like domain -accounting for about 25% of all residues-this structure is intriguingly reminiscent of the extended rod-like structure of a double-stranded RNA helix, and appears similarly suitable as a ligand for the ectodomain of TLR3 as suggested by its crystal structure.

In humans stathmin is expressed at relatively high levels in the central nervous system, testis and thymus and its expression is particularly prominent during neurogenesis and elevated in response to damage (see below). In a variety of other organs stathmin is also expressed, but at levels that are at least 10-fold lower than in the above organs. It is remarkable that the expression levels of stathmin are the highest in those vital organs that are generally recognised as immune-priviledged sites in the mammalian body and require a powerful local anti-inflammatory potential. The thymus is key in programming for central immunological tolerance for self antigens, and high expression of stathmin in this organ secures effective control of adaptive immunity against it. As a consequence of marked thymic expression of stathmin in humans, it may be expected that robust immunological tolerance exists for the molecule. Administration of stathmin to humans is therefore unlikely to provoke cellular or humoural (antibody) immune responses which is of significant advantage when considering its potential therapeutic use.

The predominance of stathmin in the nervous system is even more pronounced for the related stathmin-like proteins SCG10 (stathmin-like 2) and RB3 (stathmin-like 4) that are expressed in the human central nervous system at about 100-fold higher levels than in other organs. SCG10 in particular appears to be neuron-specific and tends to accumulate in membranes of growth cones of axons and dendrites. Several data indicate that stathmin levels in the central nervous system gradually decline with increasing age, and that trauma-induced elevated expression of stathmin becomes blunted. Also in rodents stathmin is widely expressed in the embryonic and neonatal brain while its levels of expression progressively decline during development. In the adult rat brain, immunoreactivity is only retained in highly proliferative areas such as the adult subventricular zone (SVZ) which has a particularly important role in generating glial and neural progenitors. Also in response to demyelinating conditions cells in the SVZ re-acquire this gliogenic potential. In line with its likely function in development and/or migration of glial progenitor cells stathmin is down-regulated during oligodendrocyte differentiation *in vitro* and re-expressed in oligodendrocytes in the brains of MS patients. This parallels its elevated expression in animal models of demyelination. Stathmin accumulates not only inside the oligodendrocyte cell body but also closely associated with myelin membranes. Apart from the apparent stathmin response to demyelination, increased expression of stathmin or its family members has also been documented in rats after application of experimental cortical lesions and hypoglossal nerve injury. In rat cortical neuronal cultures, stathmin is induced in response to cycloheximide-induced stress, heat stress and the glutamate antagonist MK801. In cultured oligodendrocytes stathmin expression is induced by mediators including TNF-α and TGF-β. In rat retinal ganglion cells, ciliary neurotrophic factor (CNTF) selectively induces RB3 after axotomy. Interestingly therefore, expression of stathmin and stathmin-like proteins is induced in these different neural cells at least in part by several factors such as TNF-α, TGF-β and CNTF that are produced by astrocytes in response to TLR3 activation (see below). These observations suggest the existence of a positive feedback mechanism that promotes stathmin-TLR3 interactions in the CNS: TLR3 activation on astrocytes leads to secretion of several factors that promote stathmin expression in other neural cells, while at the same time (in this case primarily through IFN-β) it also promotes TLR3 expression itself.

The pattern of stathmin expression in vertebrates along with accumulating functional data thus strongly suggest that the various members of the stathmin family play complementary roles in the development, maturation, and functional regulation in response to damage or trauma especially of the central nervous system. Inhibition of stathmin expression with antisense oligonucleotides prevents nerve growth factor-induced differentiation of rat PC 12 cells into sympathetic-like neurons. Reducing the expression of stathmin with an antisense oligonucleotide inhibits migration of new neurons from the SVZ to the olfactory bulb via the rostral migratory stream. In line with an important role of stathmin especially in the CNS, inactivation of the stathmin gene in mice leads to axonopathy in aged (20-month old) mice. Also, it has recently been shown that stathmin-deficient mice show deficits in long-term potentiation in the lateral nucleus of the amygdala, resulting in decreased memory in fear conditioning. Stathmin deficient mice additionally displayed defects in innate fear that normally occurs in naturally adverse environments. Interestingly therefore, stathmin appears to play an important role in the molecular mechanisms gouverning innate and learned fear.

Other than these effects, ablation of stathmin expression in mice does not appear to produce gross defects. Probably, the presence of various stathmin family members (in particular in the CNS) compensates for the loss of stathmin and limits the functional deficits in mice when they fail to produce only one of the four stathmin-like family members. Such redundancies do not exist in *Drosophila* in which only a single stathmin-related gene is known to exist, designated the D-stathmin gene. D-stathmin is structurally homologous to vertebrate stathmin and displays very similar molecular and biochemical properties. Also, the intron/exon junctions of the *Drosophila* stathmin gene are the same as those in vertebrates indicating common ancestry and like for the RB3 gene in vertebrates, the *Drosophila* stathmin gene encodes for several splice variants. During *Drosophila* embryogenesis, expression of D-stathmin is mainly restricted to migrating germ cells and neurons of the central and peripheral nervous system. D-stathmin expression in the early *Drosophila* embryo is similar to that of stathmin in the mouse or in *Xenopus.* D-stathmin is highly and uniformly expressed at the very beginning of embryogenesis before cellularisation, when mitosis is very active and microtubules are highly dynamic. During embryogenesis, after the end of the blastoderm stage, D-stathmin is only expressed in germ cells and cells of the central and peripheral nervous systems. This pattern that is very similar to that found for vertebrate stathmin. In *Drosophila,* inhibiting stathmin expression by siRNA leads to dramatic defects only in the nervous system. In the normal embryo, most of the axons of the ventral nerve cord are organized in a repetitive pattern of commissures. In siRNA-treated embryos on the other hand, formation of the commissures fails to occur or is defective. Also the peripheral nervous system is affected. Thus, D-stathmin is crucially important at various stages of differentiation and maturation of the *Drosophila* nervous system, mostly in ways that are relevant to neurite and axon formation.

The transcriptional and/or translational regulation of stathmin expression is only partly documented. Based on a sequence analysis of the stathmin gene, putative binding sites exist for the transcription factors E2F (position -701 to -694; -28 to -21; -19 to -11 and 715 to 722), sp1 (-123 to -114; -97 to -88, 760-769, 789-798 and 871-880) and AP-2 (-231 to -223 and -207 to -198). The regulatory role of E2F has been documented in more detail In response to genotoxic stress. E2F mediates downregulation of stathmin expression in the NIH3T3 cell line (Polager and Ginsberg, J Biol Chem 278: 1443-1449 [2003]). In the human prostate carcinoma cell line PC-3-M, the E2F sites in the stathmin gene were found essential for overexpression of the gene product. Especially E2F therefore appears to be important for controlling levels of stathmin although in different cell types, E2F-mediated regulation can apparently impact on expression levels in different ways.

Limited information is available on stathmin expression during neurodegenerative disorders in humans. In contrast to markedly elevated levels of stathmin in inflammatory demyelinating lesions that typify multiple sclerosis, levels of stathmin appear to be lower in Alzheimer's Disease-associated pathology as compared to normal brains. The difference in stathmin levels between Alzheimer's and normal brains, however, is relatively modest. Okzazaki and co-workers have reported decreased levels and altered subcellular distribution of stathmin and SCG10 only in association with tangles in Alzheimer's brains but not with plaques. Such a mild decrease has also been noted by others. Also in a mouse model of amyotrophic lateral sclerosis, stathmin levels appear lower than normal. Together, these data suggest that high levels of stathmin are induced by inflammation as seen during multiple sclerosis, and low levels of stathmin are associated with a state in which the brain's ability to appropriately respond to trauma or damage is compromised.

### Fields of application

The invention discloses that stathmin or stathmin-like proteins can act as agonists for TLR3-mediated signalling by astrocytes, and that this property can be used to activate anti-inflammatory and tissue-repair responses at a time that such is required. With regard to the CNS, this is for example the case when the damaging effects need to be counteracted of chronic or acute neurodegenerative disorders including but not limited to those described herein. Activation of the TLR3-mediated signalling pathway in astrocytes, either directly by ligation of TLR3 using stathmin or stathmin-like proteins, or indirectly by another activation step involving one of the down-stream TLR3-associated signalling molecules, will thus lead to production of a range of astrocyte-derived neuroprotective, regenerative and anti-inflammatory factors that are expected to have a beneficial effect in CNS disorders. In line with previous reports, our data also confirm that TLR3-mediated signalling will lead to induction of higher levels of expression of TLR3 itself on astrocytes. At the same time, several of the factors that are secreted in response to TLR3 activation promote the expression of stathmin and/or stathmin-like proteins. These observations support the idea that stathmin/TLR3 interactions are self promoting.

### Identifying a TLR3 agonist

At the present time versions of double-stranded RNA are known for their ability to activate TLR3-mediated signalling. Ways to identify additional compounds that can activate TLR3-mediated signalling pathways involve screening assays that monitor TLR3 signal transduction pathway in cells that express TLR either naturally, or after transfection with an TLR3-encoding gene construct. Such methods have been recently described in detail in the literature. In preferred embodiments, the readout for the screening assay is based on the use of native genes or, alternatively, transfected or otherwise artificially introduced reporter gene constructs which are responsive to the TLR3 signal transduction pathway. Reporter genes and reporter gene constructs particularly useful for the assays include, e.g., a reporter gene operatively linked to a promoter sensitive to NF-κB or IRF-3. Reporter genes operatively linked to the TLR3-sensitive promoter can include, without limitation, an enzyme (e.g., luciferase, alkaline phosphatase, β-galactosidase, chloramphenicol acetyltransferase (CAT), etc.), a bioluminescence marker (e.g., green-fluorescent protein (GFP, etc.), a surface-expressed molecule (e.g., fibroblast growth factor receptor), and a secreted molecule (e.g., CXCL8, MIP-2α, MIP-1α, TNF-α). In assays relying on enzyme activity readout, substrate can be supplied as part of the assay, and detection can involve measurement of chemiluminescence, fluorescence, colour development, incorporation of radioactive label, drug resistance, or other marker of enzyme activity. For assays relying on surface expression of a molecule, detection can be accomplished using flow cytometry (FACS) analysis or functional assays. Secreted molecules can be assayed using enzyme-linked immunosorbent assay (ELISA) or bioassays. These and other suitable readout systems are well known in the art and are commercially available. Using such assays the functional TLR3 is contacted with a test compound in order to identify a compound that activates the TLR3-mediated production of repair factors. Such a compound is a natural or synthetic compound that is capable of inducing a TLR3-mediated response when contacted with the reporter cell. Such an test compound that selectively binds to TLR3 and induce a TLR3 signal transduction pathway includes but is not limited to nucleic acids, including oligonucleotides and polynucleotides; oligopeptides; polypeptides; lipids, including lipopolysaccharides; carbohydrates, including oligosaccharides and polysaccharides; and small molecules.

Libraries of compounds that can be used as test compounds are available from various commercial suppliers, and they can be made to order using techniques well known in the art, including combinatorial chemistry techniques. Especially in combination with high throughput screening methods, such methods including in particular automated multichannel methods of screening, large libraries of test compounds can be screened according to the methods of the invention. Large libraries can include hundreds, thousands, tens of thousands, hundreds of thousands, and even millions of compounds.

Thus in preferred embodiments, the methods for screening test compounds can be performed on a large scale and with high throughput by incorporating, e.g., an array-based assay system and at least one automated or semi-automated step. For example, the assays can be set up using multiple-well plates in which cells are dispensed in individual wells and reagents are added in a systematic manner using a multiwell delivery device suited to the geometry of the multiwell plate. Manual and robotic multiwell delivery devices suitable for use in a high throughput screening assay are well known by those skilled in the art. Each well or array element can be mapped in a one-to-one manner to a particular test condition, such as the test compound. Readouts can also be performed in this multiwell array, preferably using a multiwell plate reader device or the like. Examples of such devices are well known in the art and are available through commercial sources. Sample and reagent handling can be automated to further enhance the throughput capacity of the screening assay, such that dozens, hundreds, thousands, or even millions of parallel assays can be performed in a day or in a week. Fully robotic systems are known in the art for applications such as generation and analysis of combinatorial libraries of synthetic compounds. Alternatively, the binding of a test compound to the TLR3 polypeptide can also be determined directly. For example, a radiolabelled test substance can be incubated with the TLR3 polypeptide so that binding of the test substance to the polypeptide can be monitored. Typically, the radiolabelled test substance can be incubated with cell membranes containing the polypeptide until equilibrium is reached. The membranes can then be separated from a non-bound test substance and dissolved in scintillation fluid to allow the radioactive content to be determined by scintillation counting. Non-specific binding of the test substance may also be determined by repeating the experiments in the presence of a saturating concentration of a non-radioactive ligand. Preferably, a binding curve is constructed by repeating the experiment with various concentrations of the test substance.

### Use of the TLR3-agonist

In addition to using the TLR3-agonists, such as stathmin and/or stathmin-like proteins directly, the present invention also envisages the in *vivo* promotion of the TLR3-agonists, such as stathmin and/or stathmin-like proteins. To promote the beneficial consequences of interactions between the agonist compound, such as stathmin or stathmin-like proteins, with TLR3 on astrocytes, various strategies can be envisaged. First, the agonist can be administered in such as way that they gain access to various tissues and can activate TLR3 on the surface of target cells such as fibroblast-like cells (astrocytes, synoviocytes), epithelial cells or mast cells. Under normal conditions, access of peripherally administered proteins to the CNS is very limited due to the existence of a blood-brain barrier which severely restricts passage of blood-borne substances into the brain and spinal cord parenchyma. Yet, it should be noted that in inflammatory CNS disorders activation and consequently, partial disruption of this barrier has been well documented. The activated and partially disrupted state of the blood-brain barrier under those conditions allow for "leakage" of blood-derived proteins of molecular weights far exceeding that of for instance the stathmin protein through the endothelial barrier into CNS tissue. Thus, especially in anatomical areas of ongoing inflammation in the CNS (areas where TLR3 expression is also increased and where therapeutic intervention should be directed at), access by peripheral proteins is strongly enhanced. Secondly, substances can be administered that induce higher levels of expression of the agonist such as stathmin or stathmin-like proteins notably in cells of the CNS, or that promote surface exposure or even secretion of the agonist by resident cells, thus promoting local activation of TLR3-mediated signalling. Thirdly, substances can be administered that promote formation and/or stabilise the functional structural element of the agonist for interactions with TLR3, which in the case of stathmin most likely includes the extended alpha helical structure between residues 44 and 138. Fourthly, substances can be administered that induce higher levels of expression of TLR3 on target cells such as fibroblast-like cells (astrocytes, synoviocytes), epithelial cells or mast cells. The present invention discloses that this is likely to contribute to beneficial responses. In fact, IFN-β is already an example of one such substance that has this effect and is known to act in a clinically beneficial way during multiple sclerosis. Fifthly, substances can be administered that activate selective TLR3-mediated signalling pathways in target cells such as fibroblast-like cells (astrocytes, synoviocytes), epithelial cells or mast cells.

Stathmin, stathmin-like proteins or any of the substances that promote stathmin expression, stabilisation of its helix, TLR3 expression or TLR3-mediated signalling used in aspects of the present invention may be used in non-complexed, non-bound form, i.e. in free form, not covalently linked to another (bio)molecule, but may also be used in complexed form designed for example to facilitate transport to a target tissue such as the CNS, or to enhance its biological half-life. It is also possible to use a combination of stathmin and stathmin-like proteins in aspects of the present invention.

In principle, stathmin and stathmin-like proteins of all possible sources are suitable for use in aspects and embodiments of the present invention. For instance, a suitable stathmin and stathmin-like proteins may be obtained from plants, from animals or from micro-organisms, such as yeasts. They may be produced in those sources naturally as a metabolite or may be produced therein as recombinant proteins. The stathmin and stathmin-like proteins may be isolated from these organisms or can be used in a less pure form, i.e. as an enriched fraction, or in the case of microorganisms such as (recombinant) yeasts by taking the complete organism(s) or fractions thereof. Further, stathmin and stathmin-like proteins may be isolated from other suitable sources, such as from milk, egg, soy, yeast, bacteria, algae, plants, meat, brain, etc. or may be synthetically prepared, for use in a pharmaceutical composition according to the invention or for instance in a nutraceutical composition (i.e. a food item or food supplement).

Stathmin and stathmin-like proteins may be derived from the above sources by methods known to the skilled person for instance by extraction with (organic) solvents, chromatographic separation, precipitation, crystallization and/or enzymatic of chemical hydrolysis. The isolation of stathmin or stathmin-like proteins may for instance occur by using stathmin-binding proteins.

Stathmin, stathmin-like proteins or any of the substances that promote stathmin expression, stabilisation of its helix, TLR3 expression or TLR3-mediated signalling may be formulated with standard pharmaceutically acceptable carriers and/or excipients as is routine in the pharmaceutical art. These compounds may be administered by enteral or parenteral routes such as via oral, buccal, anal, pulmonary, intra-nasal, intravenous, intra-arterial, intramuscular, intraperitoneal, transdermal, topical or other appropriate administration routes.

The skilled person is aware that therapeutic proteins/peptides are mostly administered as parenteral (injectable) preparations as a result of their poor oral bioavailability which is due to degradation by proteolytic enzymes, poor membrane permeability and large molecular size. However, the oral route would be preferred to the parenteral administration because it is more convenient for self-administration, non-invasive and more patient friendly. Consequently, efforts have intensified over the past two decades to maximize the extent of absorption of protein and peptide drugs in order to achieve optimum bioavailability via the oral route. A suitable oral delivery system retains the drug and maintains its integrity until it gets to the region of maximum absorption where the protein/peptide is released. The skilled person may perform various pharmacokinetic studies to evaluate the effect of the various routes of administration. Generally, the requirements for therapeutic proteins with respect to evaluating the pharmacokinetics of the product are the same as for conventional products, but specific considerations are needed related to the inherent characteristics of proteins. The pharmacokinetics (absorption, distribution and elimination) are preferably characterised during single-dose and steady-state conditions in relevant populations. However, the pharmacokinetic requirements may differ depending on the type of protein.

A stathmin or stathmin-like protein, or a derivative or a precursor thereof, may be provided to a subject in need thereof for prophylactic or therapeutic reasons in any administration form. For instance in the form of a food product, a food supplement, or a pharmaceutical preparation, all such administration forms being capable of initiating the TLR3-mediated repair response, whereby in particular the exertion of the neuroprotective effects of TLR3-activation are considered. The pharmaceutical preparation may be in the form of a capsule, tablet, lozenge, dragee, pill, droplet, suppository, powder, spray, vaccine, ointment, paste, cream, inhalant, patch, aerosol, as a drip, an infusion, injection, and the like. Typically, the therapeutic compositions are prepared as injectables, either as liquid solutions or suspensions; solid forms suitable for solution in, or suspension in, liquid vehicles prior to injection may also be prepared. Yet in another alternative embodiment, the stathmin or stathmin-like proteins or compositions of the invention may be administered from a controlled or sustained release matrix inserted in the body of the subject.

Further to the stathmin or stathmin-like protein, or a derivative or a precursor thereof, the pharmaceutical composition of the invention may comprise a suitable pharmaceutically acceptable carrier, and optionally additional pharmaceutically acceptable excipients

For therapeutic treatment, stathmin or stathmin-like proteins may be obtained or produced as described above and applied to the subject in need thereof. The stathmin or stathmin-like protein may be administered to a subject by any suitable route, preferably in the form of a pharmaceutical composition adapted to such a route and in a dosage that is effective for the intended treatment. Therapeutically effective dosages of the stathmin or stathmin-like protein required for treating the disorder, for instance for prevention and/or treatment of exaggerated inflammation in the body of a human or animal subject, can easily be determined by the skilled person, for instance by using animal models.

As stated above, the term "therapeutically effective amount" as used herein refers to an amount of a therapeutic, *viz.* a stathmin or stathmin-like protein, to reduce or prevent the tissue deterioration process or induce the TLR3-mediated repair response, or to exhibit a detectable therapeutic or prophylactic effect. The effect can be detected by, for example, measurement of clinical signs resulting from tissue degradation such as motor functions or behaviour, direct assessment of tissue degeneration by histological examination, or by monitoring levels of biomarkers that are associated with tissue degeneration in biological fluids or tissue samples or by any other suitable method of assessing the progress or severity of inflammation which methods are known *per se* to the skilled person. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician or experimenter. Methods that permit the clinician to establish initial dosages are known in the art. The dosages to be administered must be safe and efficacious.

Dosages for achieving the therapeutic effects of the medicament, pharmaceutical composition, food item or food supplement described herein may easily be determined by the skilled person. The dose of a compound to be administered may be determined according to various parameters, especially according to the substance used; the age, weight and condition of the patient to be treated; the route of administration; and the required regimen. A physician will be able to determine the required route of administration and dosage for any particular patient. For purposes of the present invention, an effective daily dose will be from about 0.01 µg/kg to 1 g/kg and preferably from about 0.5 µg/kg to about 400 mg of therapeutic protein/kg body weight in the individual to which it is administered. A typical daily dose is from about 0.1 to 50 mg per kg of body weight, according to the activity of the compound, the age, weight and conditions of the subject to be treated, the type and severity of the disease and the frequency and route of administration. Preferably, daily dosage levels are from 5 mg to 2 g.

In a pharmaceutical composition as described above, a stathmin or stathmin-like protein, or a derivative or a precursor thereof, is used in an amount of from 0.01 to 99.9 % by (dry) weight, preferably from 0.01 to 10 wt.%, and more preferably from 0.01 to 5 wt.%.

The present invention further relates to a method for the preparation of a pharmaceutical composition for the prevention and/or treatment of a neurodegenerative disorder selected from the group consisting of stroke, hypovolemic shock, traumatic shock, reperfusion injury, multiple sclerosis, AIDS-associated dementia, neuron toxicity, Alzheimer's disease (AD), head trauma, acute spiral cord injury, Huntington's disease (HD), Parkinson's Disease (PD) and related synucleinopathies, dystonia, Tourette Syndrome, Frontotemporal Dementias (FTDs) and related tauopathies, prion disorders such as Fatal Familial Insomnia (FFI) and other disorders related to Creutzfeldt-Jakob disease, amyotrophic lateral sclerosis (ALS), trinucleotide repeat diseases, Motor Neurone disease, progressive supranuclear palsy, REM Sleep Behavior Disorder (RBD) and cancer in a subject, comprising processing or incorporating a stathmin or stathmin-like protein, or a derivative or a precursor thereof, as an active substance, together with a pharmaceutically acceptable carrier in a pharmaceutical composition.

The preparation of a pharmaceutical composition may very suitably occur by mixing all separate ingredients such as fillers, binders, lubricants and optionally other excipients together with a stathmin or stathmin-like protein, or a derivative or a precursor thereof, and processing the mixture obtained to a pharmaceutical preparation.

A pharmaceutical composition according to the present invention may, in addition to comprising one or more stathmin or stathmin-like proteins, comprise one or more other pharmaceutically active compounds. Very suitable additional pharmaceutically active compounds are for instance additional anti-inflammatory compounds, antiviral agents, antibodies against infectious prions, and other therapeutic compounds generally used for the treatment of neurodegenerative and/or neuro-inflammatory diseases. A preferred pharmaceutical composition comprising additional pharmaceutically active compounds takes the form of a combined preparation.

The present invention in particular relates to a combined preparation of a stathmin or stathmin-like protein, or a derivative or a precursor thereof, and a medicament which is neuro-toxic, or is recognized as damaging nervous tissue. The advantage of such a combined preparation is that the harm to the nervous tissue as imparted by said medicament (i.e. by a pharmaceutical compound or by an excipient comprised in a pharmaceutical composition comprising said compound) is considerably decreased as a result of the presence of the stathmin or stathmin-like proteins in said preparation. A combined preparation may have the form of at least two separate preparations at least one preparation comprising a stathmin or stathmin-like protein and at least another preparation comprising the neuro-damaging medicament, which two separate preparations are packaged together and intended for simultaneous, separate or sequential use. Preferably, however, a combined preparation according to the present invention comprises a stathmin or stathmin-like protein and a neuro-damaging medicament together in the form of a single preparation.

The present invention thus contemplates the prevention or inhibition of drug-or drug excipient induced neuro-toxicity or nervous tissue damage in general terms, preferably arising from, but not limited to exaggerated inflammation associated with acute or chronic use of drugs. Examples of drugs and excipients that have been associated with neuro-toxicity are analgesic drugs (for example opioids), antidepressants, drugs to manage pain, psychoses or personality disorders, drugs to manage sleeping disorders, cytostatic drugs used in the management of cancer. These examples are medicaments which can cause neuro-toxic effects, often in conjunction with some level of inflammation.

In another aspect, the present invention relates to the use of a stathmin or stathmin-like protein, or a derivative or a precursor thereof, and a medicament which causes inflammation, or is recognized as enhancing inflammation, for the manufacture of a combined preparation for simultaneous, separate or sequential use in therapy.

In general terms, the stathmin-inducible TLR3-mediated response can be defined as 'wound healing", which is a complex process involving integrated steps of cell proliferation, migration, differentiation and production of extracellular matrix components. Based on the present understanding of the biological functions of the mediators induced by TLR3 activation in astrocytes and synoviocytes (as elaborated above), these mediators are supportive for the process of wound healing. Several of the factors produced by TLR3-activated astrocytes and synoviocytes have well-known repair functions in various organs and tissues other than the CNS or the joint. Therefore, application of compounds that selectively activate TLR3-mediated responses can be envisaged also for inducing wound healing outside these organs. The term "inducing" as used herein, refers to the activation, stimulation, enhancement, initiation and/or maintenance of cellular mechanisms or processes necessary for the formation of a tissue or a portion thereof, repair process or tissue development as described herein. This can be achieved in a subject by contacting a wound with an effective amount of stathmin or a stathmin-like protein or another substance that can activate TLR3-mediated responses. A subject may be any mammal, preferably human. The contacting may be topically or systemically. Examples of topical administration include, for example, contacting the wound with a lotion, salve, gel, cream, paste, spray, suspension, dispersion, hydrogel, ointment, or oil comprising stathmin, a stathmin-like protein or another TLR3 agonist. Systemic administration includes, for example, intravenous, intraperitoneal, intranasal, intramuscular injections of a composition containing stathmin, a stathmin-like protein or another TLR3 agonist. Administration of such a TLR3 activating compound may produce beneficial effects following traumatic injuries or in conditions including arthritis, osteoporosis and other musculo-skeletal disorders, burns, ulcers and other skin lesions, peripheral nerve disease and cardiovascular diseases including ischemia and atherosclerosis. Other potential tissues which can be treated by the methods and compositions of the invention include epidermal, eye, uro-genital, gastrointestinal, cardiovascular, muscle and connective tissues.

The invention may also be applied in a wide variety of other disorders where an anti-inflammatory, anti-fibrotic or regenerative response is beneficial. These disorders may include but not be limited to fibrotic disorders of various organs, allergies and inflammatory degenerative disorders of various organs including joints, gastro-intestinal tract, pancreas, skin, mucosal tissue and lungs.

### EXAMPLE

Given the emerging differences in both expression and functionality of several different TLR family members between rodents and humans, data from rodent models for CNS disorders cannot be directly extrapolated to the human situation without additional supportive evidence. To circumvent this problem we chose to study TLR functions in cultured human astrocytes obtained from post-mortem brain samples shortly after death of a donor. Such astrocytes display many morphological and functional properties (especially in the regulation of inflammatory pathways) that are indistinguishable from human astrocytes in *vivo.* Cell biological features of these cultured human adult astrocytes are thus considered sufficiently representative for their behaviour in *vivo* in an adult human brain to provide a useful conceptual framework to build on.

### Materials and methods used in example study

### Human brain samples

Human brain material was obtained via the rapid autopsy system of the Netherlands Brain Bank which supplies postmortem specimens from clinically well documented and neuropathologically confirmed cases and controls. Autopsies are performed on donors from whom written informed consent has been obtained either from the donor or direct next of kin. Samples were controlled for age, sex, seasonal alterations, circadian variation, clock time of death and medication. Postmortem factors include postmortem delay, storage time and laterality. Samples were also controlled for quality by monitoring the agonal state by pH. For all experiments, brain material from controls was used except for the experiment of Fig. 5A and 5B that was derived of a case of Parkinson's disease.

### Isolation and culture of human adult astrocytes and endothelial cells

Adult human astrocytes from post-mortem subcortical white matter samples were isolated and cultured as previously described (Bsibsi et al., J Neuropath Exp Neurol 61:1013-1021 [2002]). As determined by specific staining for cellular markers for astrocytes (glial fibrillary acidic protein; GFAP) and microglia (CD68) astrocyte cultures were essentially homogeneous; as a rule all cells expressed GFAP and none of them expressed CD68. When indicated astrocytes were stimulated with 50 µg/mL poly I:C (Amersham Pharmacia Biotech, Piscataway, NJ) or 200 ng/mL LPS (Sigma-Aldrich, Steinheim, Germany) to activate TLR3 and TLR4, respectively. These doses were selected on the basis of maximum effects in astrocytes. It should be noted that contaminants in the commercial LPS preparation could possibly lead to some level of TLR2 activation in addition to TLR4 by the LPS preparation used but not of TLR3. When stimulated with cytokines, astrocytes were supplied with TNF-α, IFN-γ, IL-β (each at 500 U/mL), IL-4 (at 250 U/mL), IL-6 (at 200 U/mL), IL-12 (at 50 ng/mL), IL-10 (at 20 ng/mL) and TGF-β (at 12.5 ng/mL) (PeproTech, Rocky Hill, NY). Control cDNA array profiling experiments included treatment with 50 µg/mL cycloheximide for 30 min prior to and during poly I:C treatment, or with 200 U/mL IFN-β (Avonex). Endothelial cells (UVEC) were isolated from umbillical cords and cultured on gelatin-coated dishes in M199 supplemented with 20nM HEPES, 10 % human serum, 10 % heat-inactivated NBCS, 150 mg/mL ECGF, 5 U/mL heparin, 100 IU/mL penicillin and 100 mg/mL streptomycin.

Proliferation of human astrocytes and human endothelial cells was determined by conversion of WST-1 by viable cells (Roche Applied Science, Indianapolis, IN) for 3 h, and quantified by determining optical density at 490 nm.

### Real-time PCR

Total cellular RNA was isolated using RNA-Bee^{™} as previously described (Bsibsi *et al.,* [2002] *supra*). Subsequently, RNA was reverse transcribed into cDNA and levels of TLR1-10 and β-actin as a reference were determined by quantitative real-time PCR. The following fluorogenic molecular beacons and primers (Biolegio, Nijmegen, The Netherlands) were used:
β-actin: sense primers: 5' GGTCATCACCATTGGCAATGA 3';
anti-sense primer 5' ACGTCACACTTCATGATGGAGTTG 3'; beacon
cgtgccGCACTCTTCCAGCCTTCCTTCCTGggcacg;
TLR1: sense primer 5' GAAGAAAGTGAATTTTTAGTTGATAGGTCA 3'; anti-sense primer
5' ACAGTGATAAGATGTCAGAAGTCCAAAG 3'; beacon:
cgtgccATCCACGTTCCTAAAGACCTATCCCAGAggcacg; TLR2: sense primer 5'
GAAATGTGAAAATCACCGATGAAAG 3'; anti-sense primer: 5'
TCCACTTTACCTGGATCTATAACTCTGTC 3'; beacon
CgtgccTTTGATGACTGTACCCTTAATGGAGTTggcacg; TLR3: sense primer 5'
CAGTACATCGAGTTCTTGGTTTCAAA 3'; anti-sense primer:
5'GAGAAATGTTCCCAGACCCAATC 3'; beacon:
CgtgccCAGACAGACAGAACAGTTTGAATATGCAGCggcacg; TLR4: sense primer 5'
TAAAGAATTTAGAAGAAGGGGTGCC 3'; anti-sense primer: 5'
CAACAATCACCTTTCGGCTTTTA 3'; beacon:
CgtgccGAGACTTTATTCCCGGTGTGGCCAggcacg.
The β-actin beacon contained 5' fluorescein VIC, and the 3'quencher Dabcyl. The TLR1-4 beacons contained 5' fluorescein FAM, and the 3'quencher Dabcyl. Thermal cycling consisted of 95 °C for 5 min and 40 cycles of 95 °C for 30 s, 56 °C for 20 s, 56 °C for 20 s and 72 °C for 30 s. PCR was performed using an ABI PRISM® 7700 sequence detection system (Applied Biosystems, Foster City, CA). Data were analyzed using sequence detector version 1.7 software.

### Organotypic brain slice cultures

Organotypic human brain slice cultures were set up and analyzed as previously described (Verwer et al., FASEB J. 16:54-60 [2002]). Briefly, tissue blocks were collected from different post-mortem cortical samples and 200-µm slices were produced. These slices were cultured for 1 week in Poly I:C- or LPS-conditioned astrocyte media that were obtained upon culturing astrocytes in the presence of either 50 µg/mL poly I:C or 200 ng/mL LPS. As a control untreated astrocyte-conditioned media from the same donor was used. All astrocyte-conditioned media were harvested after 48 h and stored at -20 °C. Viability of neurons in the slice cultures was evaluated using calcein-AM and ethidium homodimer-1 (Molecular Probes, Eugene, OR). A Zeiss 410 Invert confocal laser scanning microscope was used to inspect the slices and quantify staining.

### cDNA array profiling

Analysis of the mRNA profile of astrocytes was performed by hybrid selection of ³²P-labeled cDNA on Clontech human cytokine/chemokine Atlas® arrays as previous described (Meeuwsen et al., Glia 43:243-253 [2003]). Hybridization signals for each of the 268 cytokine, chemokine and growth factor (receptor) genes were calculated as the mean of duplicate measurements, corrected for background intensity and quantified using software provided by the manufacturer. Relative expression was calculated by dividing these hybridization signals by the mean signal for all nine housekeeping reference genes on the corresponding array.

Expression signals are expressed as the ratio of these relative levels of expression over those found in the untreated control cultures ± standard error of the mean. Gene profiling was performed using astrocytes from four different control donors.

### cDNA bank screening

Cos-7 cells (25,000 cells per well in 96-well plates) were transfected for 5 h with 768 pools of cDNA derived from human brain tumours (5 pooled tumours including meningioma, oligodendroglioma, astrocytoma grades II and IV, and medulloblastoma; NCI CGAP Brn35; Invitrogen) using lipofectamine, after which cells were thoroughly washed. Transfection with green-fluorescent protein-encoding cDNA was used as an internal control for transfection efficiencies. After continued culture of the transfected cells for 48 h, 75 µL of each culture supernatant was harvested and added to cultured human astrocytes seeded at 1,000 cells per well in 75 µL. After 48 h of culture, supernatants were harvested and evaluated for CXCL8 release using ELISA. Stimulation of astrocytes with poly I:C served as positive controls for CXCL8 release in all cases, non-transfected Cos 7 culture supernatants as negative controls.

Additional control experiments using murine astrocytes or transfected HEK293 cells were performed essentially as described previously by several other research groups.

### Statistical methods

All data in Fig. 2 are expressed as means ± standard deviations. Groups were compared by one-way analysis of variance (ANOVA), and p values were calculated by post-hoc multiple comparison tests. To evaluate statistical significance in neuronal viability in organotypic brain slice cultures the non-parametric Kruskal-Wallis test was applied to the absolute counts of viable or dead neurons in each microscopic field. P values < 0.05 were considered statistically significant.

### Results

### Preferential TLR3 induction in human astrocytes in response to cytokines or TLR agonists

The TLR expression profile of cultured adult human astrocytes derived from six different donors was examined by RT-PCR. Taking into account some donor-to-donor variation post-confluent astrocytes predominantly express TLR4 accompanied by lower levels of TLR2 and TLR3 (Fig. 1A). Expression levels of TLR1 were generally very low while in all six cultures analysed expression of TLRS-10 was essentially undetectable by RT-PCR. TLR expression levels were next compared to that of cells from the same donor activated with a range of cytokines (Fig. 1B). Following stimulation with TNF-α, IL-1β, IFN-γ, IL-12, IL-4 or IL-6 a strikingly selective induction of TLR3 of up to 360 fold was observed. The other TLR were not induced and in several cases were even suppressed which was particularly apparent for TLR2. No induction of TLR3 was seen after treatment of astrocytes with the anti-inflammatory cytokines TGF-β and IL-10. Induction of elevated levels of TLR3 expression was also observed after stimulation of astrocytes with poly I:C (a synthetic mimic of double-stranded RNA) or with LPS to activate TLR3 and TLR4, respectively (Fig. 2A). Upon stimulation with poly I:C or LPS up to 400-fold induction of TLR3, and in some cases also of TLR2 was observed while as a rule, expression of the other TLR did not change. Induction of TLR2 was an indirect effect of TLR activation by poly I:C or LPS since it could be inhibited by cycloheximide, a protein synthesis inhibitor, whereas the induction of TLR3 was insensitive to this treatment (Fig. 2B). Also upon exposure to H₂O₂ to induce oxidative stress selective induction of TLR3 was observed (data not shown).

These data extend previously published data on the restricted TLR profile of adult human astrocytes and reveal marked expression of TLR4 on cultured cells and a strikingly strong and selective induction of TLR3 upon activation by pro-inflammatory cytokines, TLR agonists or oxidative stress. This is fully in line with our previous observation that within the inflammatory environment of late stage multiple sclerosis lesions TLR3 and TLR4 are prominently present on gliotic astrocytes whereas they are undetectable by immunohistochemistry in healthy brains. Interestingly, astrocytes express TLR3 and TLR4 only on their cell surface which contrasts exclusive intracellular localization of these TLR in other cell types such as dendritic cells or microglia. These data, as summarised in Figs. 3A and 3B, are largely consistent with recent reports by other groups confirming TLR3 expression on foetal human and murine astrocytes.

### Gene profiling of human adult astrocytes stimulated with poly I:C and LPS

The dominant expression of TLR3 and TLR4 on activated astrocytes prompted us to examine the functional response mediated by these two TLR family members by gene profiling. Expression signals were evaluated by cDNA array analysis for 268 cytokines, chemokines, growth factors and their receptors that are key to the variety of astrocyte functions. Validation of these cDNA arrays in examining astrocyte responses has previously been documented by us. Astrocytes were cultured in the presence of poly I:C, LPS or both agonists together and after 48 h, gene expression profiles were compared to those in untreated parallel cultures. Figure 9 summarises the averaged data from four separate experiments, each time using cells derived from a different donor. The results reveal that poly I:C but not LPS induced a range of mediators of growth, differentiation and neuroprotection along with several chemokines, anti-inflammatory cytokines and cytokine receptors. In line with previous reports (Doyle et al., Immunity 17:251-263 [2002], Doyle et al., J. Immunol. 170:3565-3571 [2003]), mediators of type I interferon production were also induced. Only three other gene products were found to be down-regulated by at least a factor of 2. These included VEGF receptor 2 (ratio of expression 0.22), ERBB4 receptor protein-tyrosine kinase (0.43) and *Smo* (0.48). The p40 subunit of the powerful pro-inflammatory mediators IL-12 and IL-23 was down-regulated by a factor of almost 2.

Several of the gene products induced by poly I:C are well-known mediators of neuroprotection. Brain-derived neurotrophic factor for example regulates dendrite formation in cortical neurons and protects neurons from infectious damage and apoptosis. Neurotrophin-4 promotes neuronal survival, differentiation and maturation (Poo, Nat Rev Neurosci 2: 24-32 [2002]). Similarly, glial growth factors 1 and 2 and pleiotrophin support survival and differentiation of glial cells and neurons and the ephrin type-B receptor 1 guides migration and promotes connectivity of new neurons. Another mediator triggered by poly I:C is TGF-β2 which reduces virus-induced demyelination in mice, protects neurons from cell death and inhibits proliferation of astrocytes. The neuroprotective mediators ciliary neurotrophic factor and leukemia inhibitory factor that are induced by poly I:C are also well known for their ability to promote remyelination. This particular function is likely to be aided by the concomitant poly I:C-mediated downregulation with a factor of 1.96 of *Jag1.* Along with these neuroprotective and repair mediators, several growth and differentiation factors are also induced including GM-CSF, embryonic growth/differentiation factor 1 and vascular endothelial growth factor (VEGF)-C. The latter is a potent angiogenic molecule like CXCL8 that is also induced by poly I:C.

While so far most attention has been given to the ability of different TLRs to trigger pro-inflammatory cytokines, poly I:C activation of astrocytes turns out to primarily trigger potent anti-inflammatory cytokines. One of the most prominent responses to TLR3 activation in astrocytes (as well as in synoviocytes) is over 10-fold induction of tumour necrosis factor-stimulated gene 6 (TSG-6) which is a powerful anti-inflammatory product as assessed in several models of acute and chronic tissue damage, especially of arthritis. The anti-inflammatory character of the TLR3-mediated response is further supported by 7.5-fold induction of IL-9, 2.3-fold induction of IL-10 and 3.2-fold induction of IL-11, all three of which are well-known anti-inflammatory cytokines. None of these three cytokines are induced by LPS. Both poly I:C and LPS also induced TNF-α. This mediator is often regarded as a typical pro-inflammatory mediator but especially in the human CNS its functional role varies and can be anti-inflammatory as well. Concomitant with induction of these mediators poly I:C leads to almost two-fold downregulation of the p40 subunit of IL-12 and IL-23 further emphasising the overall anti-inflammatory character of the astrocytic TLR3-mediated response. Within the time span of the experiment type I interferons were not markedly induced by poly I:C but induction was seen of upstream regulators of IFN type I synthesis such as interferon consensus sequence-binding protein and interferon regulatory factor-1. This suggest that type I IFN production by astrocytes in response to TLR3 is likely to occur, but relatively late in the response. It is of interest to note the generally central role of IFN-β in TLR3 functions. Not only is IFN-β a major product released upon TLR3 signalling in several experimental cellular systems, it also induces elevated expression of TLR3 itself. At the same time, IFN-β is a therapeutic agent with proven beneficial effects in the neurodegenerative disorder multiple sclerosis. This in itself appears to support the notion that in the CNS, where astrocytes are the most abundant cells and preferentially express TLR3, a self-amplifying loop between TLR3 expression, TLR3 activation and IFN-β production forms part of a beneficial mechanism.

Among the gene products strongly induced by poly I:C as well as LPS are several chemokines including CXCL8, CXCL2 and CCL3. Poly I:C but not LPS also leads to induction of CCL4, CXCL6, CCL5, CXCL9 and CCL2 albeit to a lesser extent. Generally, the predominant functional association of chemokines is with inflammation and recruitment of leukocytes. Yet, it should be noted that for all the above chemokines that are triggered by TLR3 both astrocytes and neurons express functional receptors. This emphasises a more particular relevance of these chemokines for regulating neural cell migration and tissue remodelling within the CNS. To underpin this notion, CXCL8 and CXCL2 are also strongly induced in astrocytes by IFN-β. Together with cycloheximide treatment the impact of IFN-β was examined by cDNA arrays as a control to exclude possible indirect effects on astrocytes by TLR3-triggered mediators (data not shown). Interestingly, IFN-β is also a therapeutic agent in multiple sclerosis. Its beneficial effects on this condition would be difficult to reconcile with IFN-β-induced CXCL8 and CXCL2 predominantly acting as inflammation-promoting signals. Also for this reason, we favour the notion that the above TLR3-induced chemokines are likely to primarily regulate neural cell migration within the CNS itself rather than promote inflammation.

As is apparent from Figure 9, LPS induces a much more limited response in astrocytes and lacks the ability to activate many of the neuroprotective mediators and anti-inflammatory cytokines induced by poly I:C. Also the combination of LPS and poly I:C mimics the poly I:C-mediated stimulation of astrocytes only to a very limited extent.

### TLR3- but not TLR4-triggered mediators inhibit astrocyte growth, stimulate endothelial cell growth and promote neuronal survival

Given the multi-faceted response to TLR3 or TLR4-activation, it is difficult to assess the functional relevance of the induced mediators at face value alone. Therefore, functional assays were performed with astrocyte culture media harvested at different times following stimulation with poly I:C or LPS. Such poly I:C- and LPS-conditioned media were examined for their effects on the growth of purified astrocytes and endothelial cells. In addition, their impact was tested on neuronal viability in organotypic human brain slice cultures.

Poly I:C-conditioned astrocyte medium, but not LPS-conditioned medium inhibited astrocyte proliferation over a 3-day culture period by about 35% (p< 0.003; Fig. 4A). Since such inhibition was not observed when culture media were harvested after only 2 h, this effect cannot be attributed to residual poly I:C in the conditioned astrocyte medium but reflects the combined action of mediators secreted by the cells. Possibly, TGF-β2 and insulin growth factor-binding protein 6 (cf. Figure 9) play a role in the anti-proliferative effect given their previously documented ability to inhibit proliferation of different cell types. Astrocyte growth is a major feature of gliosis, a common response to injury in the CNS that can be regarded as a mechanism to rapidly seal off healthy tissue from a damaging insult. Yet, it results in dysfunctional scar tissue and attenuating gliosis is known to allow for improved functional recovery in animal models of CNS trauma. It should be noted that this appears not to be unique to the CNS since fibrosis (rapid and abundant growth of fibroblast-like cells to form scar tissue in response to injury) is associated with impaired recovery after trauma or damage to other tissues as well including skin, liver and lungs. Very often, reducing the extent of scar formation is considered beneficial and may allow for improved functional recovery. Thus, anti-fibrotic drugs or agents hold significant potential as therapeutic substances in a wide variety of human disorders. Given the similarity between the responses of astrocytes and synoviocytes to TLR3 activation it appears likely that the anti-fibrotic effects of the respond extends to other cell types and tissues as well suggesting that stathmin or stathmin-like proteins could serve as anti-fibrotic substances for a wide variety of applications.

Consistent with the presence in poly I:C-conditioned media of the well-known angiogenic mediators VEGF-C and CXCL8 (cf. Figure 9), these media significantly promoted growth of human endothelial cells over a 3-day period (p< 0.003; Fig. 4B). These growth-promoting effects were similar to those of recombinant fibroblast growth factor-2 that was used as a positive control. Clearly, angiogenesis in the CNS can be considered a beneficial response to injury. Especially in the CNS, optimal blood supply in order to secure sufficient influx of nutrients and oxygen is of key importance.

Finally, poly I:C-and LPS-conditioned media were tested for their effects on neuronal survival in organotypic human brain slice cultures. Such cultures were maintained for 1 week and neuronal survival was evaluated with calcein-AM and ethidium homodimer-1. This revealed significantly improved survival of neurons in slice cultures supplied with poly I:C-conditioned medium but not LPS-conditioned medium (p< 0.03; Fig. 5). Culturing organotypic human brain slices in control medium with freshly added poly I:C promoted neuronal survival equally well (Fig. 5C). This suggests that also in the context of such slices local astrocytes can be activated by poly I:C to produce neuroprotective mediators during culture, and that any TLR3 activation on other neural cell types does not interfere with this protection.

The major conclusion from the above data is that astroglial TLR3 mediates a response that is distinct from TLR4 and involves production of a variety of neuroprotective factors, angiogenic factors, chemokines and anti-inflammatory cytokines. Consistent with the well-known properties of many of these TLR3-mediated products the combined collection of mediators produced by astrocytes in culture upon TLR3 activation inhibit astrocyte growth, stimulate endothelial cell growth and, importantly, promote survival of neurons in organotypic brain slice cultures. None of these effects were observed using the collection of TLR4-triggered mediators from astrocyte culture supernatants. Such a beneficial and largely neuroprotective quality of the TLR3-mediated response in astrocytes appears to be in line with the observation that astroglial TLR3 expression in MS lesions is particularly prominent at late stage lesions, and induced at least in cultured astrocytes by pro-inflammatory mediators that are known to be present in an active lesion. Thus, it appears more likely that TLR3 on astrocytes (but not microglia) plays a role in promoting repair as a follow-up to inflammation rather intensifying ongoing inflammatory reactions. Also it is striking that especially IFN-β, known for its beneficial effects in MS, plays a key role in signalling by TLR3 much more so than by other TLR. IFN-β release and subsequent IFN-β-induced TLR3 expression represents an important self-amplifying element of the TLR3-mediated response in many cell types. In astrocytes the production of IFN-β in response to TLR3 activation is probably a slow response as it did not reach significant levels during the 48-h culture period monitored in the present study. We did, however, observe induction of factors that may be expected to eventually induce type I interferon production.

### Stathmin acts as novel TLR3 agonists

The above data thus indicate that abundant TLR3 expression is induced on human astrocytes as a response to inflammatory mediators, and that TLR3-mediated signalling activates a powerful anti-inflammatory response, and one that promotes neuroprotection, repair and regenerative processes. In sharp contrast to the selective and strong induction of TLR3 on the surface of activated astrocytes, the only known agonist for TLR3, viz. double-stranded RNA, is remarkably scarce as an extracellular substance in the CNS. Since also for other reasons a role of double-stranded RNA as the only TLR3 agonist can be rationalised in the present context only with difficulty we decided to examine the possibility that additional TLR3 agonist molecules could be present in the human CNS itself. The existence of such an agonist would be consistent with the possibility of a TLR3-mediated endogenous repair mechanism that could be activated following an inflammatory episode or other forms of tissue damage or trauma.

A cDNA bank derived from 5 pooled tumours including meningioma, oligodendroglioma, astrocytoma grades II and IV and medulloblastoma was used as a source of sequences potentially encoding an endogenous protein agonist for TLR3. The cDNAs were cloned into plasmid vectors promoting expression and secretion of the cDNA-encoded product by mammalian cells, and these plasmids were transfected into COS-7 cells and evaluated in a two-step approach. First, pools of 50-100 cDNA-containing plasmids were transfected together and such pools (768 in total) were screened for their ability to instruct COS-7 cells to produce and secrete proteins that trigger a response in human astrocytes consistent with TLR3 activation. To monitor such an astrocyte response in this first stage, secretion of CXCL8 was evaluated since this is a sensitive marker for TLR3 activation in these and other cells (cf. Figs. 9 and 10). In murine astrocytes, IL-6 is used as a marker for TLR3-mediated responses since there is no murine CXCL8 ortholog. It should be kept in mind, however, that CXCL8 or IL-6 release is not specific for TLR3 activation: a variety of other stimuli and signalling pathways can also mediate CXCL8 or IL-6 secretion by astrocytes. When CXCL8 secretion by human astrocytes in response to the products produced by COS-7 cells was observed, the individual plasmids contained in that pool were tested individually on their ability to encode for a product that induces CXCL8 secretion. In this way, two candidate sequences were identified that -when transcribed by COS-7 cells- led to the production of proteins that triggered marked CXCL8 secretion in astrocytes (Fig. 6). One of the two candidate cDNAs (clone C11) was identified as a product irrelevant to the current invention since it was found to act through TLR2 in follow-up experiments. The other CXCL8-inducing product (clone F5) was identified as an amino acid sequence identical to residues 43-149 of human stathmin (Figure 11).

To verify that the partial stathmin sequence encoded by the selected cDNA is indeed the CXCL8 inducing factor derived from the corresponding COS-7 transfected cells, the CXCL8 inducing effect was evaluated of purified recombinant human stathmin from a commercial source. This experiment (Fig. 7A) confirmed that at doses between 5 and 100 µg/mL, also recombinant human stathmin was capable of inducing marked CXCL8 release by cultured human astrocytes from all four donors tested. Control experiments using TLR3-directed small interfering RNA to block expression of TLR3 in astrocytes revealed that the stathmin-induced response in human astrocytes was largely -in one case completely- inhibited by such selective interference with TLR3 expression (Fig. 7B). Additional control experiments (Figs. 7C and 7D) using HEK293 cells stably transfected with individual TLR-encoding plasmids verified that the recombinant stathmin used for these experiments did not contain levels of endotoxins that allowed for activation of TLR2 or TLR4 under these conditions. In a crucial experiment it was observed that astrocytes from TLR3-deficient mice failed to respond to recombinant stathmin, like they failed to respond to poly I:C, whereas their ability to respond to activation via either TLR2 by zymosan, tigggering an only very mild response, or TLR4 by LPS was retained (Fig. 7E). These observations provide crucial evidence that the stathmin-induced response is fully TLR3-dependent.

### The extended unphosphorylated alpha helix of the conserved stathmin-like domain is likely to be the structural element binding to the TLR3 ectodomain

Combining the above novel data with information previously reported strongly suggests that the structural part of stathmin that is essential for ligation to TLR3 is the 95-amino acid long alpha helical structure between residues 44 and 138. Importantly, the TLR3-activating protein product encoded by clone F5 identified in the cDNA bank screen contains the helix-forming residues 43-149, but not the N-terminal portion 1-42 of stathmin. No less than 25% of the residues contained in this helix are negatively charged amino acids, predominantly glutamic acid residues. These render the (conserved) stathmin-like helical domain structurally reminiscent of the phosphate-lined helical structure of double-stranded RNA. Recent modelling of the ectodomain of TLR3 is consistent with the idea that an extended rod-like structure containing abundant negative charges is a crucial structural requirement for TLR3 ligation and dimerisation that are essential for signalling.

The N-terminal part of stathmin is a target of phosphorylation that is known to regulate its interaction with tubulin. Four serine residues at positions 16, 25, 38 and 63 are known to become phosphorylated by different kinases. Only residue 63 is contained in the functional F5 sequence 43-149 but additional data suggest that it is highly unlikely that phosphorylation at this only remaining site is a requirement for TLR3 agonist activity. By structural as well as functional analyses of a truncated variant protein containing resides 40-140, it has previously been demonstrated that phosphorylation of serine at position 63 almost completely disrupts the alpha helical structure of stathmin. As explained above, it is this helical structure that is likely to be essential for TLR3 ligation implying that a functional form of stathmin would include an unphosphorylated Ser-63 rather than a phosphorylated form of this residue. The studies by Steinmetz and colleagues have also clarified that as purified recombinant proteins, stathmin or truncated forms of stathmin containing the helix-forming domain are structurally ordered into such an alpha helix only for about 40-60% when examined in buffers similar to culture media. This renders it likely that under experimental conditions used by us only at best about half the protein molecules may be expected to be functional in binding to the TLR3 ectodomain. This negatively affects the apparent dose-response relationship between stathmin and TLR3-activation. As is illustrated in Figure 13, the stathmin-like proteins stathmin, SCG-10, SCLIP and RB3 all share marked sequence homologies in the region that defines the alpha helix in the conserved stathmin-like domain, including the high number of negatively charged amino acids. This is consistent with the notion that all four family members are highly likely to also share their ability to act as TLR3 agonists.

## Claims

1. A method for the treatment and/or prophylaxis of a degenerative inflammatory process in a tissue of a subject, said method comprising increasing the activity of Toll-like receptor 3 (TLR3) in cells of said tissue.

2. Method according to claim 1, wherein said method comprising administering to said subject:
- a therapeutically effective amount of at least one TLR3-agonist;
- a therapeutically effective amount of at least one compound that promotes *in vivo* expression of a TLR3-agonist;
- a therapeutically effective amount of at least one compound that promotes TLR3 expression, and/or
- a therapeutically effective amount of at least one compound that promotes TLR3-mediated signalling.

3. Method according to claim 1 or 2, wherein said subject is a mammal, preferably a human.

4. Method according to any one of claims 1-3, wherein said tissue is nervous tissue, preferably brain tissue and/or synovial tissue.

5. Method according to any one of the preceding claims, wherein said cells are glial cells, preferably astrocytes, and/or synovial cells, preferably synoviocytes.

6. Method according to any one of the preceding claims, wherein said degenerative inflammatory process is the result of a chronic or acute neurodegenerative disorder.

7. Method according to claim 6, wherein said neurodegenerative disorder is selected from the group consisting of stroke, hypovolemic shock, traumatic shock, reperfusion injury, multiple sclerosis, AIDS-associated dementia, neuron toxicity, Alzheimer's disease (AD), head trauma, acute spiral cord injury, Huntington's disease (HD), Parkinson's Disease (PD) and related synucleinopathies, dystonia, Tourette Syndrome, Frontotemporal Dementias (FTDs) and related tauopathies, prion disorders such as Fatal Familial Insomnia (FFI) and other disorders related to Creutzfeldt-Jakob disease, amyotrophic lateral sclerosis (ALS), trinucleotide repeat diseases, Motor Neurone disease, progressive supranuclear palsy, REM Sleep Behavior Disorder (RBD), and cancer.

8. Method according to any one of claims 5-7, wherein said treatment is accompanied by stimulation of a neuroprotective response in cells of said nervous tissue and/or wherein said treatment is accompanied by stimulation of a repair functions in cells of said synovial tissue.

9. Method according to any one of claims 2-8, wherein said at least one TLR3-agonist is selected from the group consisting of:
- stathmin, TLR3-activating derivatives thereof and precursors thereof;
- stathmin-like proteins, TLR3-activating derivatives thereof and precursors thereof, and
- proteinaceous compounds comprising a polypeptide chain having an amino acid sequence similarity of at least 60% with an amino acid sequence selected from the group consisting of the amino acid sequences of stathmin (SEQ ID NO: 1), SCG-10 (SEQ ID NO:2), SCLIP (SEQ ID NO: 3) and RB3 (SEQ ID NO: 4).

10. Method according to claim 9, wherein said amino acid sequence similarity relates to the stathmin-like alpha helix domain corresponding to residues 44 to 138 of SEQ ID NO: 1.

11. Method according to claim 9, wherein said stathmin-like protein is SCG10, SCLIP and/or RB3.

12. Method according to any one of claims 9-11, wherein said method further comprising the use of said TLR3-agonist in combination with compounds that promote stabilisation of the stathmin-like alpha helix domain corresponding to residues 44 to 138 of SEQ ID NO: 1.

13. A pharmaceutical composition for the treatment and/or prophylaxis of a degenerative inflammatory process in a tissue of a subject, wherein said composition comprises a therapeutically effective amount of at least one compound capable of increasing the activity of Toll-like receptor 3 (TLR3) in cells of said tissue; and b) a pharmaceutically acceptable carrier.

14. Composition according to claim 13, wherein said at least one compound is:
- a TLR3-agonist;
- a compound that promotes in *vivo* expression of a TLR3-agonist;
- a compound that promotes TLR3 expression, and/or
- a compound that promotes TLR3-mediated signalling.

15. Composition according to claim 13 or 14, wherein said subject is a mammal, preferably a human.

16. Composition according to any one of claims 13-15, wherein said tissue is nervous tissue, preferably brain tissue and/or synovial tissue.

17. Composition according to any one of claims 13-16, wherein said cells are glial cells, preferably astrocytes, and/or synovial cells, preferably synoviocytes.

18. Composition according to any one of claims 13-17, wherein said degenerative inflammatory process is the result of a chronic or acute neurodegenerative disorder.

19. Composition according to claim 18, wherein said neurodegenerative disorder is selected from the group consisting of stroke, hypovolemic shock, traumatic shock, reperfusion injury, multiple sclerosis, AIDS-associated dementia, neuron toxicity, Alzheimer's disease (AD), head trauma, acute spiral cord injury, Huntington's disease (HD), Parkinson's Disease (PD) and related synucleinopathies, dystonia, Tourette Syndrome, Frontotemporal Dementias (FTDs) and related tauopathies, prion disorders such as Fatal Familial Insomnia (FFI) and other disorders related to Creutzfeldt-Jakob disease, amyotrophic lateral sclerosis (ALS), trinucleotide repeat diseases, Motor Neurone disease, progressive supranuclear palsy, REM Sleep Behavior Disorder (RBD) and cancer.

20. Composition according to any one of claims 17-19, wherein said compound is capable of stimulating a neuroprotective response in cells of said nervous tissue and/or wherein said compound is capable of stimulating repair functions in cells of said synovial tissue.

21. Composition according to any one of claims 14-20, wherein said at least one TLR3-agonist is selected from the group consisting of:
- stathmin, TLR3-activating derivatives thereof and precursors thereof,
- stathmin-like proteins, TLR3-activating derivatives thereof and precursors thereof, and
- proteinaceous compounds comprising a polypeptide chain having an amino acid sequence similarity of at least 60% with an amino acid sequence selected from the group consisting of the amino acid sequences of stathmin (SEQ ID NO: 1), SCG-10 (SEQ ID NO:2), SCLIP (SEQ ID NO: 3) and RB3 (SEQ ID NO: 4).

22. Composition according to claim 21, wherein said amino acid sequence similarity relates to the stathmin-like alpha helix domain corresponding to residues 44 to 138 of SEQ ID NO: 1.

23. Composition according to claim 21, wherein said stathmin-like protein is SCG10, SCLIP and/or RB3.

24. Composition according to any one of claims 21-23, wherein said composition further comprising compounds that promote stabilisation of the stathmin-like alpha helix domain corresponding to residues 44 to 138 of SEQ ID NO: 1.

25. A TLR3-agonist selected from the group consisting of:
- stathmin, TLR3-activating derivatives thereof and precursors thereof,
- stathmin-like proteins, TLR3-activating derivatives thereof and precursors thereof, and
- proteinaceous compounds comprising a polypeptide chain having an amino acid sequence similarity of at least 60% with an amino acid sequence selected from the group consisting of the amino acid sequences of stathmin (SEQ ID NO: 1), SCG-10 (SEQ ID NO:2), SCLIP (SEQ ID NO: 3) and RB3 (SEQ ID NO: 4),
for use as a medicament.

26. The use of a TLR3-agonist selected from the group consisting of:
- stathmin, TLR3-activating derivatives thereof and precursors thereof,
- stathmin-like proteins, TLR3-activating derivatives thereof and precursors thereof, and
- proteinaceous compounds comprising a polypeptide chain having an amino acid sequence similarity of at least 60% with an amino acid sequence selected from the group consisting of the amino acid sequences of stathmin (SEQ ID NO: 1), SCG-10 (SEQ ID NO:2), SCLIP (SEQ ID NO: 3) and RB3 (SEQ ID NO: 4),
for the preparation of a medicament for the treatment and/or prophylaxis of a degenerative inflammatory process in a tissue of a subject.

27. The method of any one of claims 1- 12, which is for preventing or retarding the onset of a neurodegenerative disorder in a subject, in the absence of any disorder, disease, or injury.

28. The composition of any one of claims 13- 24, which is for preventing or retarding the onset of a neurodegenerative disorder in a subject, in the absence of any disorder, disease, or injury.


### Annex to the application documents - subsequently filed sequences listing

SEQUENCE LISTING

<110> Nederlandse Organisatie voor
      Toegepast-Natuurwetenschappelijk Onderzoek TNO

<120> Methods for treating neurodegenerative disorders and compositions
       for use therein

<130> P67889EP00

<140> EP 06075265.6
<141> 2006-02-03

<160> 22

<170> PatentIn version 3.3

<210> 1
<211> 149
<212> PRT
<213> Human


<220>
<221> MISC_FEATURE
<222> (44)..(138)
<223> Stathmin-like alpha helix domain

<400> 1

Met Ala Ser Ser Asp Ile Gln Val Lys Glu Leu Glu Lys Arg Ala Ser
1               5                   10                  15


Gly Gln Ala Phe Glu Leu Ile Leu Ser Pro Arg Ser Lys Glu Ser Val
            20                  25                  30


Pro Glu Phe Pro Leu Ser Pro Pro Lys Lys Lys Asp Phe Ser Leu Glu
        35                  40                  45


Glu Ile Gln Lys Lys Leu Glu Ala Ala Glu Glu Arg Arg Lys Ser His
    50                  55                  60


Glu Ala Glu Val Leu Lys Gln Leu Ala Glu Lys Arg Glu His Glu Lys
65                  70                  75                  80


Glu Val Leu Gln Lys Ala Ile Glu Glu Asn Asn Asn Phe Ser Lys Met
                85                  90                  95


Ala Glu Glu Lys Ile Thr His Lys Met Glu Ala Asn Lys Glu Asn Arg
            100                 105                 110


Glu Ala Gln Met Ala Ala Lys Leu Glu Arg Leu Arg Glu Lys Asp Lys
        115                 120                 125


His Ile Glu Glu Val Arg Lys Asn Lys Glu Ser Lys Asp Pro Ala Asp
    130                 135                 140


Glu Thr Glu Ala Asp
145

<210>  2
<211>  179
<212>  PRT
<213>  Human

<400>  2

Met Ala Lys Thr Ala Met Ala Tyr Lys Glu Lys Met Lys Glu Leu Ser
1               5                   10                  15

Met Leu Ser Leu Ile Cys Ser Cys Phe Tyr Pro Glu Pro Arg Asn Ile
            20                  25                  30

Asn Ile Tyr Thr Tyr Asp Asp Met Glu Val Lys Gln Ile Asn Lys Arg
        35                  40                  45

Ala Ser Gly Gln Ala Phe Glu Leu Ile Leu Lys Pro Pro Ser Pro Ile
    50                  55                  60

Ser Glu Ala Pro Arg Thr Leu Ala Ser Pro Lys Lys Lys Asp Leu Ser
65                  70                  75                  80

Leu Glu Glu Ile Gln Lys Lys Leu Glu Ala Ala Glu Glu Arg Arg Lys
                85                  90                  95

Ser Gln Glu Ala Gln Val Leu Lys Gln Leu Ala Glu Lys Arg Glu His
            100                 105                 110

Glu Arg Glu Val Leu Gln Lys Ala Leu Glu Glu Asn Asn Asn Phe Ser
        115                 120                 125

Lys Met Ala Glu Glu Lys Leu Ile Leu Lys Met Glu Gln Ile Lys Glu
    130                 135                 140

Asn Arg Glu Ala Asn Leu Ala Ala Ile Ile Glu Arg Leu Gln Glu Lys
145                 150                 155                 160

Glu Arg His Ala Ala Glu Val Arg Arg Asn Lys Glu Leu Gln Val Glu
                165                 170                 175

Leu Ser Gly


<210>  3
<211>  180
<212>  PRT
<213>  Human

<400>  3

Met Ala Ser Thr Ile Ser Ala Tyr Lys Glu Lys Met Lys Glu Leu Ser
1               5                   10                  15

Val Leu Ser Leu Ile Cys Ser Cys Phe Tyr Thr Gln Pro His Pro Asn
            20                  25                  30

Thr Val Tyr Gln Tyr Gly Asp Met Glu Val Lys Gln Leu Asp Lys Arg
        35                  40                  45

Ala Ser Gly Gln Ser Phe Glu Val Ile Leu Lys Ser Pro Ser Asp Leu
    50                  55                  60

Ser Pro Glu Ser Pro Met Leu Ser Ser Pro Pro Lys Lys Lys Asp Thr
65                  70                  75                  80

Ser Leu Glu Glu Leu Gln Lys Arg Leu Glu Ala Ala Glu Glu Arg Arg
                85                  90                  95

Lys Thr Gln Glu Ala Gln Val Leu Lys Gln Leu Ala Glu Arg Arg Glu
            100                 105                 110

His Glu Arg Glu Val Leu His Lys Ala Leu Glu Glu Asn Asn Asn Phe
        115                 120                 125

Ser Arg Gln Ala Glu Glu Lys Leu Asn Tyr Lys Met Glu Leu Ser Lys
    130                 135                 140

Glu Ile Arg Glu Ala His Leu Ala Ala Leu Arg Glu Arg Leu Arg Glu
145                 150                 155                 160

Lys Glu Leu His Ala Ala Glu Val Arg Arg Asn Lys Glu Gln Arg Glu
                165                 170                 175

Glu Met Ser Gly
            180

<210>  4
<211>  216
<212>  PRT
<213>  Human

<400>  4

Met Thr Leu Ala Ala Tyr Lys Glu Lys Met Lys Glu Leu Pro Leu Val
1               5                   10                  15

Ser Leu Phe Cys Ser Cys Phe Leu Ala Asp Pro Leu Asn Lys Ser Ser
            20                  25                  30

Tyr Lys Tyr Glu Gly Trp Cys Gly Arg Gln Cys Arg Arg Lys Asp Glu
        35                  40                  45

Ser Gln Arg Lys Asp Ser Ala Asp Trp Arg Glu Arg Arg Ala Gln Ala
    50                  55                  60

Asp Thr Val Asp Leu Asn Trp Cys Val Ile Ser Asp Met Glu Val Ile

65                  70                  75                  80

Glu Leu Asn Lys Cys Thr Ser Gly Gln Ser Phe Glu Val Ile Leu Lys
                85                  90                  95

Pro Pro Ser Phe Asp Gly Val Pro Glu Phe Asn Ala Ser Leu Pro Arg
            100                 105                 110

Arg Arg Asp Pro Ser Leu Glu Glu Ile Gln Lys Lys Leu Glu Ala Ala
        115                 120                 125

Glu Glu Arg Arg Lys Tyr Gln Glu Ala Glu Leu Leu Lys His Leu Ala
    130                 135                 140

Glu Lys Arg Glu His Glu Arg Glu Val Ile Gln Lys Ala Ile Glu Glu
145                 150                 155                 160

Asn Asn Asn Phe Ile Lys Met Ala Lys Glu Lys Leu Ala Gln Lys Met
                165                 170                 175

Glu Ser Asn Lys Glu Asn Arg Glu Ala His Leu Ala Ala Met Leu Glu
            180                 185                 190

Arg Leu Gln Glu Lys Asp Lys His Ala Glu Glu Val Arg Lys Asn Lys
        195                 200                 205

Glu Leu Lys Glu Glu Ala Ser Arg
    210                 215

<210>  5
<211>  21
<212>  DNA
<213>  Artificial

<220>
<223>  beta-actin sense primer

<400>  5
ggtcatcacc attggcaatg a                                                21

<210>  6
<211>  24
<212>  DNA
<213>  Artificial

<220>
<223>  beta-actin anti-sense primer

<400>  6
acgtcacact tcatgatgga gttg                                             24

<210>  7
<211>  36
<212>  DNA
<213>  Artificial

<220>
<223>  beta-actin beacon

<400>  7
cgtgccgcac tcttccagcc ttccttcctg ggcacg                                36


<210>  8
<211>  30
<212>  DNA
<213>  Artificial

<220>
<223>  TLR1 sense primer

<400>  8
gaagaaagtg aatttttagt tgataggtca                                       30


<210>  9
<211>  28
<212>  DNA
<213>  Artificial

<220>
<223>  TLR1 anti-sense primer

<400>  9
acagtgataa gatgtcagaa gtccaaag                                         28


<210>  10
<211>  40
<212>  DNA
<213>  Artificial

<220>
<223>  TLR1 beacon

<400>  10
cgtgccatcc acgttcctaa agacctatcc cagaggcacg                            40


<210>  11
<211>  25
<212>  DNA
<213>  Artificial

<220>
<223>  TLR2 sense primer

<400>  11
gaaatgtgaa aatcaccgat gaaag                                            25


<210>  12
<211>  29
<212>  DNA
<213>  Artificial

<220>
<223>  TLR2 anti-sense primer

<400>  12
tccactttac ctggatctat aactctgtc                                        29


<210>  13

<211>  39
<212>  DNA
<213>  Artificial

<220>
<223>  TLR2 beacon

<400>  13
cgtgcctttg atgactgtac ccttaatgga gttggcacg                                39


<210>  14
<211>  26
<212>  DNA
<213>  Artificial

<220>
<223>  TLR3 sense primer

<400>  14
cagtacatcg agttcttggt ttcaaa                                              26


<210>  15
<211>  23
<212>  DNA
<213>  Artificial

<220>
<223>  TLR3 anti-sense primer

<400>  15
gagaaatgtt cccagaccca atc                                                 23


<210>  16
<211>  42
<212>  DNA
<213>  Artificial

<220>
<223>  TLR3 beacon

<400>  16
cgtgcccaga cagacagaac agtttgaata tgcagcggca cg                            42


<210>  17
<211>  25
<212>  DNA
<213>  Artificial

<220>
<223>  TLR4 sense primer

<400>  17
taaagaattt agaagaaggg gtgcc                                               25


<210>  18
<211>  23
<212>  DNA
<213>  Artificial

<220>
<223>  TLR4 anti-sense primer

<400>  18

caacaatcac ctttcggctt tta 23

<210> 19
<211> 36
<212> DNA
<213> Artificial

<220>
<223> TLR4 beacon

<400> 19
cgtgccgaga ctttattccc ggtgtggcca ggcacg 36

<210> 20
<211> 840
<212> DNA
<213> Artificial

<220>
<223> Clone F5 - cDNA clone - Nucleotide sequence

<220>
<221> misc_feature
<222> (1)..(48)
<223> Plasmid sequence

<400> 20
ggctggtccg gtccggaatt cccggggaat cgtcgaccca cgcgtccgcc cacgcgtccg 60

ggatctttcc ctggaggaaa ttcagaagaa attagaagct gcagaagaaa gacgcaagtc 120

ccatgaagct gaggtcttga agcagctggc tgagaaacga gagcacgaga aagaagtgct 180

tcagaaggca atagaagaga acaacaactt cagtaaatgg cagaagagaa actgacccac 240

aaaatggaag ctaataaaga gaaccgagag gcacaaatgg ctgccaaact ggaacgtttg 300

cgagagaagg ataagcacat tgaagaagtg cggaagaaca aagaatccaa agaccctgct 360

gacgagactg aagctgacta atttgttctg agaactgact ttctccccat ccccttccta 420

aatatccaaa gactgtactg gccagtgtca ttttattttt tccctcctga caaatatttt 480

agaagctaat gtaggactgt ataggtagat ccagatccag actgtaagat gttgttttag 540

gggctaaagg ggagaaactg aaagtgtttt actctttttc taaagtgttg gtctttctaa 600

tgtagctatt tttcttgttg catcttttct acttcagtac acttggtgta ctgggttaat 660

ggctagtact gtattggctc tgtgaaaaca tatttgtgaa aagagtatgt gtggcttctt 720

ttgaactgtt agatgctgaa tatctgttca cttttcaatc ccaattctgt cccaatctta 780

ccagatgcta ctggacttga atggttaata aaactgcaca gtgctaaaaa aaaaaaaaaa 840

<210> 21
<211> 149
<212> PRT
<213> Human

<400> 21

Met Ala Ser Ser Asp Ile Gln Val Lys Glu Leu Glu Lys Arg Ala Ser
1               5                   10                  15

Gly Gln Ala Phe Glu Leu Ile Leu Ser Pro Arg Ser Lys Glu Ser Val
            20                  25                  30

Pro Glu Phe Pro Leu Ser Pro Pro Lys Lys Lys Asp Leu Ser Leu Glu
        35                  40                  45

Glu Ile Gln Lys Lys Leu Glu Ala Ala Glu Glu Arg Arg Lys Ser His
    50                  55                  60

Glu Ala Glu Val Leu Lys Gln Leu Ala Glu Lys Arg Glu His Glu Lys
65                  70                  75                  80

Glu Val Leu Gln Lys Ala Ile Glu Glu Asn Asn Asn Phe Ser Lys Met
                85                  90                  95

Ala Glu Glu Lys Leu Thr His Lys Met Glu Ala Asn Lys Glu Asn Arg
            100                 105                 110

Glu Ala Gln Met Ala Ala Lys Leu Glu Arg Leu Arg Glu Lys Asp Lys
        115                 120                 125

His Ile Glu Glu Val Arg Lys Asn Lys Glu Ser Lys Asp Pro Ala Asp
    130                 135                 140

Glu Thr Glu Ala Asp
145

<210>  22
<211>  106
<212>  PRT
<213>  Artificial

<220>
<223>  Clone F5 - Protein sequence

<400>  22

Asp Leu Ser Leu Glu Glu Ile Gln Lys Lys Leu Glu Ala Ala Glu Glu
1               5                   10                  15

Arg Arg Lys Ser His Glu Ala Glu Val Leu Lys Gln Leu Ala Glu Lys
            20                  25                  30

Arg Glu His Glu Lys Glu Val Leu Gln Lys Ala Ile Glu Glu Asn Asn
        35                  40                  45

Asn Phe Ser Lys Met Ala Glu Glu Lys Leu Thr His Lys Met Glu Ala
    50                  55                  60

Asn Lys Glu Asn Arg Glu Ala Gln Met Ala Ala Lys Leu Glu Arg Leu
65                  70                  75                  80

Arg Glu Lys Asp Lys His Ile Glu Glu Val Arg Lys Asn Lys Glu Ser
                85                  90                  95

Lys Asp Pro Ala Asp Glu Thr Glu Ala Asp
            100                 105